# EUROPEAN PATENT APPLICATION

(11) **EP 4 000 626 A1**
(43) Date of publication of application: **25.05.2022**
(21) Application number: 20207806.9
(22) Date of filing: 16.11.2020
(51) Int. Cl.: A61K 36/28, A61K 31/198, A61K 31/047, A61K 31/4375, A61K 31/366, A61K 31/045, A61K 36/06, A61K 36/899, A23L 33/105, A23L 33/125, A23L 33/14, A23L 33/17, A61P 3/00, A61P 3/02, A61P 3/04, A61P 5/24, A61P 15/02, A61P 15/12, A61P 3/10

(54) **PHARMACEUTICAL PREPARATION**

(71) Applicant: concrete flowers GmbH, 2603 Felixdorf (AT)
(72) Inventor: Seiler, Friederike, 1040 Wien (AT)

(57) **Abstract**

A multi-component composition, a multiphase product comprising the composition, methods of improving fertility, sexual desire and insulin resistance, specifically fertility problems related to insulin resistance, by administration of the composition, and use thereof for the treatment of diseases and disorders, specifically diseases and disorders of the glucose metabolism, such as insulin resistance, and/or the female reproductive system, such as e.g. polycystic ovary syndrome (PCOS) and reduced sexual function. Said composition comprising L-citrullin and silymarin and either Mixture A comprising myo-inositol and D-chiro-inositol, or Mixture B comprising berberine, monacolin K and policosanol.

## Description

### FIELD OF THE INVENTION

The present invention relates to a multi-component composition, a multiphase product comprising the composition, methods of improving fertility, sexual desire and insulin resistance, specifically fertility problems related to insulin resistance, by administration of the composition, and use thereof for the treatment of diseases and disorders, specifically diseases and disorders of the glucose metabolism, such as insulin resistance, and/or the female reproductive system, such as polycystic ovary syndrome (PCOS), and reduced sexual function.

### BACKGROUND OF THE INVENTION

A majority of women and adolescent girls with PCOS have insulin resistance with consequent hyperinsulinemia. The latter commonly leads to hyperandrogenemia with negative effects on reproductive function. The American Diabetes Association reports that high glucose levels increase the risk of early miscarriage in pregnancy by 30-60%. Insulin resistance not only affects women already diagnosed with metabolic syndrome but very commonly manifests through abnormalities related to the menstrual cycle, i.e. excessive bleeding, chronic anovulation, mastodynia or polycystic ovaries. Paralleling the marked increase in obesity in the last 30-40 years, insulin-induced hyperandrogenemia is already relatively common in 60-70% of pre and post pubertal girls with marked obesity (BMI >90-95%ile for age). Additional evidence shows that low sex hormone binding globulin (SHBG) levels, hyperandrogenemia and elevated insulin predict subsequent development of the metabolic syndrome and class 3 obesity by the middle of the third decade (Glueck CJ, et al., Sex hormone-binding globulin, oligomenorrhea, polycystic ovary syndrome, and childhood insulin at age 14 years predict metabolic syndrome and class III obesity at age 24 years. J Pediatrics. 2011; 159:308-313). This provides support to the concept that all women with insulin resistance (defined as elevated IFG, IGT, LDL levels and metabolic syndrome) should receive the earliest possible treatment.

Polycystic ovary syndrome (PCOS) is a common endocrinopathy with anovulation and hyperandrogenism as cardinal symptoms. In recent years it has been recognized that insulin resistance is an intrinsic feature of the disease and plays a central role in its pathogenesis. PCOS is associated with significant reproductive morbidity, as demonstrated by the high prevalence of anovulatory infertility, spontaneous abortion, gestational diabetes and pre-eclampsia. The association of insulin resistance with this reproductive pathology is well documented. Increased insulin levels, a side effect of obesity, cause the pituitary gland to release large amounts of hormones that interfere with ovulation.

In PCOS, several hormonal disorders reinforce each other in a hormonal control loop. One of the basic endocrinological constellations - possibly the beginning of the syndrome - is the increased release of the hormone LH and a reduced release of the hormone FSH from the pituitary gland. The LH stimulates the production of male hormones, from which female hormones are then produced in a second step. In PCO syndrome, the basally elevated LH level leads to an increase in male sex hormones. These male hormones are converted (flavoured) into female hormones (oestrogens) and released in the opposite direction to the normal menstrual cycle - i.e. acyclically. This disrupts the normal cycle of the "fertility clock" and is virtually a self-sustaining process: due to the continuously high LH levels in the cycle, the egg cells are confronted at a too early stage with the hormone LH, which is responsible, among other things, for triggering ovulation. They remain immature - i.e. they cannot mature to the normal size of an egg follicle (approx. 25 mm) - and therefore ovulation occurs less frequently.

The sometimes reduced responsiveness of insulin to the blood sugar level in PCO syndrome (insulin resistance) compensates for the increased insulin release of the body. The resulting elevated insulin levels reinforce the already existing surplus of male hormones in that insulin has an LH-like activity and thus further triggers the already elevated LH levels. Insulin also inhibits the formation of the binding protein SHGB in the liver, which is important for reducing male hormones.

Treatments for PCOS commonly use strong ovulation-inducing agents, which are considered effective, but have a high possibility of causing serious side effects such as multiple pregnancy or ovarian hyperstimulation syndrome (OHSS), and thus cause difficult problems. Furthermore, there are significant problems in the treatment for unmarried females or young females. Many of these patients are reluctant to consult obstetricians or gynecologists and are often left without receiving proper medical attention. Such patients will be in danger where symptoms such as hypertrophy of the ovary membrane or the like will further progress and, thus, the treatment thereof becomes even more difficult. Also, even when unmarried or young female patients consult obstetricians or gynecologists, both the doctors and the patients tend to avoid, as much as possible, treatment using strong agents such as an ovulation-inducing agent or gonadotropin. Also, the administration of oral contraceptive agents is often not welcomed by the patients. Accordingly, the patients are often diagnosed as having "irregular menstruation" or the like to receive only temporary relief against the symptoms. Thus, conventional methods for treating polycystic ovary syndrome (PCOS) are often unsatisfactory for patients.

Glucose metabolism abnormalities, even at their early stages, may result in sexual dysfunction in women and may be associated with depressive symptoms, the severity of which depends on the degree of insulin resistance. Women with diabetes are characterized by multidimensional impairment of sexual function, while in prediabetic women this dysfunction is limited to impairment of sexual desire and sexual satisfaction. In addition to disturbances in sexual functioning, carbohydrate abnormalities are associated with depressive symptoms, more pronounced in women with diabetes than prediabetes. The total FSFI score inversely correlates with the overall BDI-II score, suggesting the existence of a relationship between the degree of sexual dysfunction and depression severity. Beyond sexual functioning, the BDI-II score is also regulated by other factors, particularly by being overweight and obesity pre-sent in many participants of our study. This hypothesis may be supported by the presence of a correlation between the BDI-II score and body mass index in weight-matched control subjects with normal glucose homeostasis. Indirect evidence exists on the correlation of insulin-sensitivity and sexuality: studies show reduced circulating levels of hormones that play a central role in the regulation of female sexual functioning, such as prolactin, testosterone and dehydroepiandrosterone sulphate, after insulin-sensitizing treatment. Elevated prolactin levels are associated with multidimensional impairment of sexual functioning in women and a reduction in its level may improve the female sexual cycle, sexual satisfaction and dyspareunia. In turn, androgen deficiency seems to induce impairment of sexual function, libido, well-being and energy, as well as may contribute to reduced cognitive functions.

Women with polycystic ovary syndrome receiving insulin-sensitizing therapy were more satisfied with their sex life, reported higher frequencies of sexual intercourse, felt less pain during sexual intercourse and reported a lowered impact of excessive body hair on sexuality than before treatment. Women with diabetes had a lower total FSFI score, as well as lower scores for all domains: sexual desire, sexual arousal, lubrication, orgasm, sexual satisfaction and dyspareunia, and these scores correlated with a degree of insulin resistance. Women with prediabetes had lower scores for sexual desire and sexual satisfaction.

There is increasing evidence that the endocrinologic and metabolic abnormalities in PCOS may have complex effects on the endometrium, contributing to the infertility and endometrial disorders observed in women with this syndrome. Androgen receptors and steroid receptor co-activators are over-expressed in the endometrium of women with PCOS. Also, biomarkers of endometrial receptivity to embryonic implantation are decreased. In addition to being responsive to the steroid hormones estradiol, progesterone, and androgens, the endometrium is also a target for insulin, the receptor for which is cyclically regulated in normo-ovulatory women. In vitro, insulin inhibits the normal process of endometrial stromal differentiation (decidualization). At physiological levels insulin likely plays a homeostatic role for energy metabolism in the endometrium, and in hyperinsulinemic states insulin action on the endometrium may activate cellular mitosis via the MAPK pathway and thus may predispose the endometrium to hyperplasia and/or cancer. The actions of insulin on the endometrium in vivo are difficult to distinguish from the actions of androgens because hyperandrogenism and hyperinsulinemia are positively correlated in women with anovulation.

In addition, insulin-like growth factors (IGFs) and their binding proteins are regulated in and act on endometrial cellular constituents, and hyperinsulinemia down-regulates hepatic IGFBP-1, resulting in elevated free IGF-I in the circulation. Thus, elevated estrogen (without the opposing effects of progesterone in the absence of ovulation), hyperinsulinemia, elevated free IGF-I and androgens, and obesity all likely contribute to endometrial dysfunction, infertility, increased miscarriage rate, endometrial hyperplasia, and endometrial cancer common in women with PCOS.

Recent studies have reported that patients affected by PCOS have a seven-fold risk of developing diabetes complications, when compared to unaffected individuals. As is well known, diabetes is a serious disease which could result in myocardial infarction, cerebral stroke, renal insufficiency, loss of eyesight caused by retinopathy, or the like. Accordingly, it is necessary that patients with PCOS be treated at a stage before diabetes complications develop.

The oral contraceptive pill (OC) has been recommended for >30 years to treat women with PCOS (Ehrmann, 2005). Its beneficial effects include reducing acne, improving hirsutism and correcting oligo-menorrhea (Ehrmann, 2005). Less well understood is the effect of oral contraceptives on the metabolic risk profile in PCOS. Studies (Rimm et al., 1992; Watanabe et al., 1994) in the general population have linked oral contraceptives, composed of 30-50 mg of ethinyl estradiol (EE), to reduced glucose tolerance and insulin resistance (Rimm et al., 1992; Watanabe et al., 1994). The link between oral contraceptives and dysglycemia is unclear with low-dose oral contraceptives (Chasen-Taber et al., 1997), but the risks reported in the general population may underestimate the risk for women with PCOS. Dyslipidemia has been linked to the progestogen component of oral contraceptives (Ball et al., 1990; Van Rooijen et al., 2002). If longterm use of oral contraceptives can induce IR, dysglycemia and dyslipidemia in the general population, then women with PCOS may have a greater risk for adverse metabolic outcomes when exposed to OCs. Previous studies have shown, that the use of oral contraceptives is significantly associated with an increase in triglycerides and a strong association between fasting glucose/ insulin ratio and oral contraceptives as well as an association between oral contraceptive use and HDL-C of women with a higher average BMI. We suspect, that in women with pre-existing PCOS despite administration of oral contraceptives insulin resistance persists, thus leading to increased triglyceride levels and low DHEAS levels. Therefore, there is an unmet therapeutic need to treat insulin resistance in women diagnosed with PCOS while administering oral contraceptives.

There is an unmet need in the field for a therapeutic agent for insulin-resistance associated female health disorders, which exhibits few, if any, side effects, is effective at inducing ovulation and restoring sexual desire and is safely available to not only females who desire to bear children, but also for unmarried or young females or peri- and postmenopausal women.

### SUMMARY OF THE INVENTION

It is the objective of the present invention, to provide a composition capable of improving female fertility and sexual function, specifically to improve symptoms commonly associated with polycystic ovary syndrome (PCOS), such as a decreased ovulation rate and reduced libido.

The problem is solved by the present invention.

It has been found that a combination of micronutrients, plant-based active ingredients, and optionally synbiotics significantly reduces insulin resistance. It has further been found that the combination of micronutrients, plant-based active ingredients and synbiotics is capable of significantly improving female fertility. Furthermore, said combination improves the ovulation rate in PCOS patients, specifically by reducing insulin resistance.

Surprisingly, the inventors also found a clear correlation between libido/ sexual desire, DHEAS and insulin resistance in women with PCOS.

During the reproductive years, testosterone in women is produced by the ovaries and by peripheral conversion of androstenedione and dehydroepiandrosterone (DHEAS), which are pre-androgens synthesised by the ovaries and adrenal glands. The pre-androgens contribute about 50% of circulating testosterone in premenopausal women. Concentrations of testosterone first begin to increase in girls at about the age of 6-8 years, when maturation of the adrenal zona reticularis results in increased production of DHEAS and its sulphate, DHEASS heralding the onset of adrenarche. Cyclical production of testosterone by the ovaries starts with the onset of ovulation; concentrations peak mid-cycle and remain high during the luteal phase.

Results of a community-based study (Davis SR et.al. JAMA 2005; 294: 91-96) of 1021 randomly recruited healthy women showed a direct association between an endogenous level of DHEASS below the tenth percentile and low sexual responsiveness in women aged 45 years or older. In women aged 18-44 years, concentrations of DHEASS below the tenth percentile were directly associated with low sexual desire, arousal, and responsiveness.

Recent studies (Christodoulaki C. et.al. J Family Reprod Health. 2017; 11(1):24-29) have revealed a significant negative correlation of DHEAS-S with the mean volume of the right and left ovary and with the maximum volume of the largest ovary in women with PCOS. Thus, low libido and sexual function, resulting from low DHEAS levels, can be explained with hyper-stimulation of ovaries related to insulin resistance in women with PCOS. We conclude, that treatment of insulin resistance in women with clinical or subclinical PCOS will lead to an improvement in libido and sexual function. According to the invention, there is provided a composition comprising L-citrullin and silymarin and
a. Mixture A comprising:
   i. myo-inositol;
   ii. D-chiro-inositol;
   or
b. Mixture B comprising:
   i. berberine;
   ii. monacolin K;
   iii. policosanol.

Specifically, L-citrullin is provided as the pure chemical compound, e.g. synthetically produced.

Specifically, silymarin is provided as the pure chemical compound, e.g. synthetically produced, or as plant extract, preferably as an extract of the milk thistle *Silybum marianum (L.) Gaertn.*

Specifically, myo-inositol is provided as the pure chemical compound, e.g. synthetically produced.

Specifically, d-chiro-inositol is provided as the pure chemical compound, e.g. synthetically produced.

Specifically, berberine is provided as the pure chemical compound, e.g. synthetically produced, or as plant extract, preferably as a dry extract of *Berberis, Berberis aristate, Berberis vulgaris, Mahonia aquifolium, Hydrastis canadensis, Xanthorhiza simplicissima, Phellodendron amurense, Coptis chinensis, Tinospora cardifolia, Argemone mexicana,* and/or *Eschscholzia californica.* Specifically, the composition comprises berberine chloride, or berberine hydrochloride.

Specifically, monacolin K is provided as the pure chemical compound, e.g. synthetically produced, or in the form of red yeast rice. According to a specific embodiment, the composition comprises red yeast rice standardized to comprise at least 10% monacolin, preferably 15% monacolin.

Specifically, policosanol is provided in synthetic form, as the pure chemical compound, or as an isolate from sugar cane, beeswax, and/or cereal grains.

According to one specific embodiment, the composition provided herein comprises Mixture A comprising 3.900 to 4.100 mg myo-inositol, preferably about 4.000 mg myo-inositol, 30 to 50 mg D-chiro-inositol, preferably about 40 mg D-chiro-inositol, 100 to 140 mg silymarin, preferably about 120 mg silymarin, and 400 to 750mg L-citrullin, preferably about 500 mg.

According to another specific embodiment, the composition provided herein comprises Mixture B comprising 400 to 700 mg berberine, preferably 550 to 650 mg and most preferably 600 mg berberine, 200 to 250 mg red yeast rice, preferably about 220 mg red yeast rice, or 10 to 40 mg monacolin K, preferably about 30 mg monacolin K, 10 to 20 mg policosanol, preferably about 12 mg policosanol, 100 to 140 mg silymarin, preferably about 120 mg silymarin, and 400 to 750mg L-citrullin, preferably about 500 mg.

According to a further specific embodiment, the composition provided herein comprises one or more pharmaceutically acceptable carriers as further described herein.

Specifically, the composition provided herein further comprises a *Mixture C* comprising any one or more of vitamin B1, vitamin B2, vitamin B6, vitamin B12, vitamin B4, vitamin E, selenium, zinc, manganese, copper, folate or folic acid, potassium iodide and L-citrullin and silymarin
and
a. Mixture A comprising:
   i. myo-inositol;
   ii. D-chiro-inositol;
   or
b. Mixture B comprising:
   i. berberine;
   ii. red yeast rice;
   iii. policosanol.

Specifically, vitamin B1 is added to the composition as the pure chemical compound, *e.g.* synthetically produced.

Specifically, vitamin B2 is added to the composition as the pure chemical compound, *e.g.* synthetically produced.

Specifically, vitamin B6 is added to the composition as the pure chemical compound, *e.g.* synthetically produced.

Specifically, vitamin B12 is added to the composition as the pure chemical compound, *e.g.* synthetically produced.

Specifically, vitamin B4 is added to the composition as the pure chemical compound, *e.g.* synthetically produced.

Specifically, vitamin E is added to the composition as the pure chemical compound, *e.g.* synthetically produced.

Specifically, selenium is added to the composition as the pure chemical compound, *e.g.* synthetically produced.

Specifically, zinc is added to the composition as the pure chemical compound, e.g. synthetically produced.

Specifically, manganese is added to the composition as the pure chemical compound, *e.g.* synthetically produced.

Specifically, copper is added to the composition as the pure chemical compound, e.g. synthetically produced.

Specifically, folate or folic acid is added to the composition as the pure chemical compound, *e.g.* synthetically produced.

Specifically, potassium iodide is added to the composition as the pure chemical compound, *e.g.* synthetically produced.

Specifically, the composition comprises any one or more of thiamine mononitrate (vitamin B1), Riboflavin 5'-monophosphate sodium salt (vitamin B2), pyridoxal 5'-phosphate monohydrate (vitamin B6), hydroxocobalamin acetate (vitamin B12), choline bitartrate (vitamin B4), D-alpha-tocopherylacetate (vitamin E), selenomethionine, zinc orotate, manganese-(II)-gluconate-dihydrate, copper bisglycinate, potassium iodide and L-5-methyltetrahydrofolate.

Further specifically, the composition comprises any one or mote of 0,8-1,4mg thiamine mononitrate, preferably 1,1mg; 0,85-1,25mg Riboflavin 5'-monophosphate sodium salt, preferably 1,05mg; 0,85-1,25mg pyridoxal 5'-phosphate monohydrate, preferably 1,05mg; 0,0015-0,0035mg hydroxocobalamin acetate, preferably 0,0025mg; 60-120mg choline bitartrate, preferably 82,5mg; 6-12mg D-alpha-tocopherylacetate, preferably 9mg; 0,02-0,06mg selenomethionine, preferably 0,04mg; 5-10mg zinc orotate, preferably 7,5mg; 1-2,5mg manganese-(II)-gluconate-dihydrate, preferably 1,5mg; 0,25-1,25mg copper bisglycinate, preferably 0,75mg; 0.3-0,7mg L-5-methyltetrahydrofolate, preferably 0.5 mg; and 0.4-1 mg potassium iodide, preferably 0.7 mg ("Mixture C").

According to a specific embodiment, the composition provided herein further optionally comprises a Mixture E comprising Vitex agnus-castus extract, black cohosh extract, and 5-L-Hydroxytryptophan.

Specifically, Vitex agnus-castus is added to the composition as the pure chemical compound, e.g. synthetically produced agnuside, or it is included in the composition as plant extract, preferably as an extract of *Vitex agnus-castus.*

Specifically, Black cohosh extract is added to the composition as the pure chemical compound, *e.g.* synthetically produced triterpene glycosides, or are included in the composition as plant extract, preferably as an extract of *Cimicifuga racemosa.*

Specifically, 5-L-Hydroxytryptophan is added to the composition as the pure chemical compound, *e.g.* synthetically produced, or is included in the composition as plant extract, preferably as an extract of *Griffonia simplicifolia.*

Specifically, the composition described herein, comprising Mixture A or Mixture B and Mixture C, and optionally comprising Mixture E comprising 8-16mg Vitex agnus-castus, preferably 12mg, 4-10 mg Black cohosh extract, preferably about 7 mg Black cohosh extract, 50 to 200 mg 5-L-Hydroxytryptophan, preferably about 100 mg.

According to a further specific embodiment, the composition provided herein optionally comprises a Mixture D comprising of active probiotic strains *L. crispatus, L. gasseri, L. jensenii, L. reuteri* and *L. rhamnosus* and pomegranate extract.

Specifically, active probiotic strains will be added to the composition freeze-dried with 50 billion colon-forming units (cfu) per gram, cfu referring to the ability of one cell to reproduce until it forms a colony.

Specifically, pomegranate extract is added to the composition as plant extract, preferably as an extract of *Punica granatum.*

Specifically the composition comprises 100 mg to 300 mg *Punica granatum* extract, preferably 200 mg, 8 to 15 mg of *L. crispatus* (50bn cfu/g), preferably 12mg, 8 to 15 mg of *L. gasseri* (50bn cfu/g), preferably 12mg, 8 to 15 mg of *L. jensenii* (50bn cfu/g), preferably 12mg, 8 to 15 mg of *L. reuteri* (50bn cfu/g), preferably 12mg, and 8 to 15 mg of *L. rhamnosus* (50bn cfu/g), preferably 12mg.

Specifically, the composition provided herein is formulated in the form of a powder, preferably water-soluble powder, provided herein is in the form of a powder, preferably water-soluble powder, a soft gel capsule, a liquid capsule, a HPMC capsule, or a chewable tablet.

Further provided herein is the composition described herein for use in the treatment of a disease or disorder. Specifically, the disease or disorder is selected from the group consisting of polycystic ovary syndrome (PCOS), insulin-resistance, obesity, dyslipidemia, coronary heart disease, metabolic syndrome, menopause, PMS (premenstrual syndrome), difficulties with ovulation, weight gain, and increased appetite.

Specifically provided herein is Mixture B for use in the treatment of polycystic ovary syndrome (PCOS), insulin-resistance, obesity, dyslipidemia, coronary heart disease, metabolic syndrome, menopause, PMS (premenstrual syndrome), difficulties with ovulation, weight gain, and increased appetite. Specifically, Mixture B is used to treat PCOS or to improve at least one parameter associated with reduced fertility, such as ovulation rate and/or insulin-resistance.

Specifically, further provided herein is Mixture A for use in the treatment of polycystic ovary syndrome (PCOS), insulin-resistance, obesity, dyslipidemia, coronary heart disease, metabolic syndrome, menopause, PMS (premenstrual syndrome), difficulties with ovulation, weight gain, and increased appetite. Specifically, Mixture A is used to improve at least one parameter associated with fertility, such as ovulation rate, libido and/or insulin-resistance.

Further provided herein is a dietary supplement, comprising the composition described herein. Specifically, the dietary supplement provided herein is in the form of a powder, preferably water-soluble powder, a soft gel capsule, a liquid capsule, a HPMC capsule, or a chewable tablet.

Further described herein is the use of the dietary supplement provided herein to reduce insulin-resistance, to increase the rate of ovulation, or for restoring sexual desire and improve FSFI.

Specifically, the dietary supplement described herein is provided for the non-therapeutic nutrition of a human subject. Specifically, the dietary supplement described herein is provided for the non-therapeutic nutrition of a human female subject to improve libido, fertility and/or ovulation rate. Specifically, the dietary supplement described herein is provided for the non-therapeutic nutrition of a human female subject to improve insulin resistance.

The dietary supplement provided herein may further be used to reduce menopause-associated symptoms such as insulin resistance, polyphagia, vaginal dryness, weight gain, reduced libido, and/or hormonal-related mood swings.

Further provided herein is a pharmaceutical preparation consisting of a number of separately packaged and individually removable daily dose units placed in a packaging unit and intended for oral administration for a period of at least 28 consecutive days, wherein said daily dose units comprise a composition comprising L-citrullin and silymarin and
a. Mixture A comprising:
   i. myo-inositol;
   ii. D-chiro-inositol;
   or
b. Mixture B comprising:
   i. berberine;
   ii. red yeast rice;
   iii. policosanol.

Further provided herein is a multiphase product for enhanced female fertility, comprising at least 28 consecutive daily dose units comprising Mixture C comprising any one or more of vitamin B1, vitamin B2, vitamin B6, vitamin B12, vitamin B4, vitamin E, selenomethionine, zinc histidine, manganese, and copper bisglycinate. Specifically, the composition comprises any one or more of thiamine mononitrate (vitamin B1), Riboflavin 5'-monophosphate sodium salt (vitamin B2), pyridoxal 5'-phosphate monohydrate (vitamin B6), hydroxocobalamin acetate (vitamin B12), choline bitartrate (vitamin B4), manganese-(II)-gluconate-dihydrate, D-alpha-tocopherylacetate (vitamin E), and
L-citrullin, silymarin and
a. Mixture A comprising:
   i. myo-inositol;
   ii. D-chiro-inositol;
   or
b. Mixture B comprising:
   i. berberine;
   ii. red yeast rice; and
   iii. policosanol.

According to a specific embodiment of the multiphase product described herein, Mixture D comprising pomegranate extract and the active probiotic strains *L. crispatus, L. gasseri, L. jensenii, L. reuteri* and *L. rhamnosus* will be administered between day 6 to day 17 of the menstrual cycle, preferably from day 8 to day 14.

Specifically, the dietary supplement provided herein is in the form of a powder, preferably water-soluble powder, a soft gel capsule, a liquid capsule, a HPMC capsule, or a chewable tablet.

Specifically, the patient or subject in need thereof is a human suffering from or suspected to suffer from polycystic ovary syndrome (PCOS), difficulties with ovulation, anovulation, insulin-resistance, oligomenorrhea, amenorrhea, or difficulties with conception.

### FIGURES

Fig. 1 shows treatment regimen for Examples 1 and 2, using the multiphase product described herein.
Fig. 2 shows treatment regimen for Example 3 and Example 4.

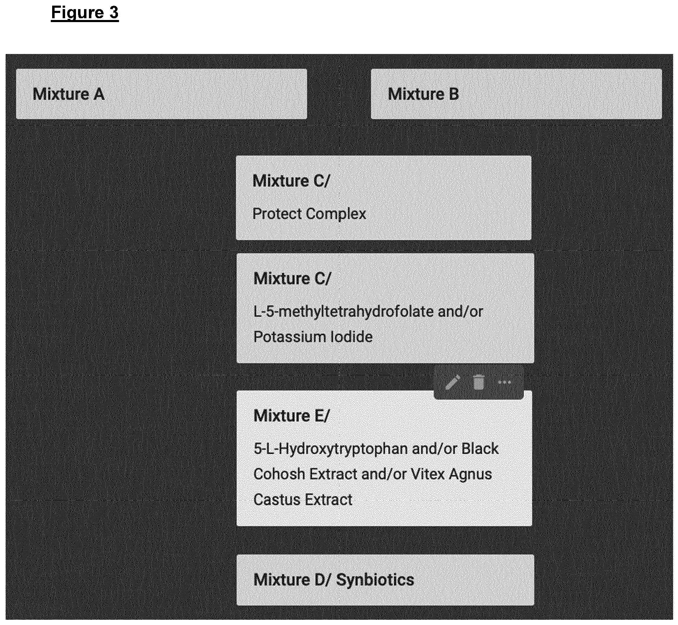

Fig. 3 shows the structure of the invention.

### DETAILED DESCRIPTION

Unless indicated or defined otherwise, all terms used herein have their usual meaning in the art, which will be clear to the skilled person. Specific terms as used throughout the specification have the following meaning.

Unless indicated otherwise, the term "about" as used herein refers to the same value or a value differing by up to +/-5 % of the given value.

To provide a more concise description, some of the quantitative expressions given herein are not qualified with the term "about". It is understood that whether the term "about" is used explicitly or not, every quantity given herein is meant to refer to the actual given value, and it is also meant to refer to the approximation to such given value that would reasonably be inferred based on the ordinary skill in the art, including approximations due to the experimental and/or measurement conditions for such given value.

The terms "comprise", "contain", "have" and "include" as used herein can be used synonymously and shall be understood as an open definition, allowing further members or parts or elements. "Consisting" is considered as a closest definition without further elements of the consisting definition feature. Thus "comprising" is broader and contains the "consisting" definition.

As used herein, the term "subject" or "individual" or "patient" shall refer to a warm-blooded mammalian, particularly a human being.

The term "patient" includes mammalian, specifically human, subjects that receive either prophylactic or therapeutic treatment or are at risk of or diagnosed of a specific disease or disorder, particularly those conditions as further described herein.

As well as proving to be effective in improving fertility and balancing hormonal levels in women affected by insulin-resistance, the compositions according to the invention are useful in treating and/or in preventing insulin resistance, metabolic syndrome, dyslipidaemia, type-2 diabetes mellitus, hirsutism and/or cardio-cerebrovascular diseases.

Specifically, the compositions of the present invention are useful to increase ovulation rates, improve hormonal parameters, such as restoration of normal LH secretion, improve the regularity of the menstrual cycle, decrease symptoms of premenstrual syndrome, reduce blood glucose levels, reduce total and LDL cholesterol in individuals with mild to moderate dyslipidemia, or moderate cardiometabolic risk, reduce androgen levels, with reductions in testosterone, reduce circulating glucose and insulin levels as well as for reducing systolic blood pressure, decrease of free IGF-I, reduction of endometrial thickness, fasting glucose, triglycerides, LDL and fasting insulin.

The compositions also find application in Medically Assisted Procreation (MAP) with a view to improving oocyte quality, optimising ovarian stimulation protocols; in particular, to prevent ovarian hyperstimulation syndrome and to improve oocyte quality. Further beneficial effects have been observed on the classic symptoms of the menopause: irritability, hypertension, osteoporosis, dyslipidemia, weight gain, hot flushes and aging of the skin.

### Mixture A

A composition comprising Mixture A, as described herein, comprises myo-inositol, d-chiro-inositol, L-citrullin and silymarin.

As used herein, "milk thistle" is a term used in its broadest sense and encompasses whole herb (fresh or dehydrated, including seed, flower, fruit, leaf, stem, or root portions), herbal preparations, herbal extracts, herbal mixtures and chemicals derived from the group of plants commonly known as milk thistle. The silymarin used in the composition described herein may be derived specifically from *Silybum marianum* and its sub-species and/or varieties. In particular implementations, pharmaceutical compositions derived from *Silybum marianum* may include preparations standardized on the basis of a family of compounds known as silymarin. As used herein, "silymarin" is a term used in its broadest sense and may refer to silymarin in many different chemical forms including silymarin and silymarin derivates. Specifically, the composition described herein includes milk thistle standardized to contain between about 10% and about 95% silymarin. In particular implementations, milk thistle standardized to contain about 80% silymarin may be used in the composition described herein.

Silymarin may be provided by any suitable source. For example, silymarin can be purchased commercially, provided as plant extract from milk thistle, or obtained from chemical synthesis.

Specifically, the composition described herein comprises at least about 90, 95, 100, 105, 110, 115, 120, 125, 130, 135, 140, 145, or 150 mg silymarin. Specifically, the composition described herein comprises at least about 100, 105, 110, 115, 120, 125, 130, 135, 140, 145, 150, 155, 160, 165, 170, 175, 180, 185, 190, 195 or 200 mg milk thistle standardized to about 80 to 95 % silymarin, preferably standardized to 90 to 95 % silymarin.

As used herein, L-citrullin is a ubiquitous amino acid, that is strongly related to arginine (Arg), and has scope beyond that normally associated with an amino acid. Cit rullin supplementation is more efficacious than that of its precursor, Arginin. Circulating Citrullin is in fact a masked form of Arginin to avoid degradation of Citrullin in the liver (Citrullin is readily taken up by liver and degraded). Thus, Citrullin is actually a better method of getting supplemental Arginine into the blood than Arginine itself, proving that Citrullin is a novel therapeutic agent in conditions of Arginine and NO deficiencies. Nitric Oxide (NO) plays pivotal roles in many physiological functions which are compromised in PCOS. The high levels of androgen and advanced glycation end products (AGEs) in PCOS women induces oxidative stress and inflammation. Recent reports show strong involvement of oxidative stress and endothelial dysfunction in PCOS pathogenesis. Both endothelial dysfunction and oxidative stress are prognostic indicators of cardio vascular disorders, pre-eclampsia, placenta-related defects and recurrent pregnancy loss. Endothelial function is compromised under PCOS, endothelial impairment being majorly attributed to endothelial nitric oxide (NO) synthesis, its bioavailability and oxidant-antioxidant mechanisms.

Specifically, the composition described herein comprises about 300, 350, 400, 450, 475, 500, 525, 550, 575, 600, 625, 650, 675, 700, 725 or 750 mg L-citrullin. L-citrullin may be provided by any suitable source. For example, L-citrullin can be purchased commercially, or obtained from chemical synthesis.

Inositol is a glucose isomer, also called a sugar alcohol, hexahydric alcohol, and vitamin B8. Inositol is involved in a number of functions, including cell membrane formation, cell growth, morphogenesis, cytoskeleton rearrangements, proliferation regulation, intracellular signaling, peripheral nerve development and function, osteogenesis, and reproduction. In cells, inositol is present both in free form and as a structural element of membrane phosphatidylinositols. The epimerization of six hydroxyl groups causes that the inositol has nine isomeric forms, with myo-inositol (MI) and D-(+)-chiro-inositol (DCI) being the most important. Myo-inositol is a phosphatidylinositol cycle precursor and a component of phospholipids and cell membranes. In addition, MI is a precursor in the synthesis of secondary hormonal transmitters such as gonadotropin-releasing hormone (gonadoliberin, GnRH), Thyroid-stimulating hormone (TSH), and insulin.

Use of MI and DCI combinations have been described for the treatment of patients suffering from PCOS (EP3424497A1 and Unfer V et al. Updates on the myoinositol plus D-chiro-inositol combined therapy in polycystic ovary syndrome. Expert Rev Clin Pharmacol. 2014;7(5): 623-31), but the inventors of the present invention have found that using a composition comprising MI and DCI in combination with silymarin and L-citrullin exhibits significant benefits, such as e.g. improved fertility in women suffering from insulin-resistance and its potential to be used as a dietary supplement.

According to a preferred embodiment, myo-inositol and D-chiro-inositol are used at a ratio of 50:1 or lower, for example, at a ratio ranging from 40:1 to 10:1. Preferably, myo-inositol is included in the composition at a weight of about 1.400, 1.450, 1.500, 1.550, 1.600, 1.650, 1.700, 1.750, 1.800, 1.850, 1.900, 1.950, 2.000, 2.050, 2.100, 2.150 or 2.200 mg. Preferably, D-chiro-inositol is included in the composition at a weight of about 30, 35, 40, 45, 50, 55, 60, 65, 70, 75, 80, 85, 90, 95 or 100 mg.

Myo-inositol and D-chiro-inositol, may be provided by any suitable source. For example, they can be purchased commercially, or obtained from chemical synthesis.

### Mixture B

A composition comprising Mixture B as described herein comprises berberine, monacolin K, policosanol , L-citrullin and silymarin.

As used herein, "milk thistle" is a term used in its broadest sense and encompasses whole herb (fresh or dehydrated, including seed, flower, fruit, leaf, stem, or root portions), herbal preparations, herbal extracts, herbal mixtures and chemicals derived from the group of plants commonly known as milk thistle. The silymarin used in the composition described herein may be derived specifically from *Silybum marianum* and its sub-species and/or varieties. In particular implementations, pharmaceutical compositions derived from *Silybum marianum* may include preparations standardized on the basis of a family of compounds known as silymarin. As used herein, "silymarin" is a term used in its broadest sense and may refer to silymarin in many different chemical forms including silymarin and silymarin derivates. Specifically, the composition described herein includes milk thistle standardized to contain between about 10% and about 95% silymarin. In particular implementations, milk thistle standardized to contain about 80% silymarin may be used in the composition described herein.

Silymarin may be provided by any suitable source. For example, silymarin can be purchased commercially, provided as plant extract from milk thistle, or obtained from chemical synthesis.

Specifically, the composition described herein comprises at least about 90, 95, 100, 105, 110, 115, 120, 125, 130, 135, 140, 145, or 150 mg silymarin. Specifically, the composition described herein comprises at least about 100, 105, 110, 115, 120, 125, 130, 135, 140, 145, 150, 155, 160, 165, 170, 175, 180, 185, 190, 195 or 200 mg milk thistle standardized to about 80 to 95 % silymarin, preferably standardized to 90 to 95 % silymarin.

As used herein, L-citrullin is a ubiquitous amino acid, that is strongly related to arginine (Arg), and has scope beyond that normally associated with an amino acid. Cit rullin supplementation is more efficacious than that of its precursor, Arginin. Circulating Citrullin is in fact a masked form of Arginin to avoid degradation of Citrullin in the liver (Citrullin is readily taken up by liver and degraded). Thus, Citrullin is actually a better method of getting supplemental Arginine into the blood than Arginine itself, proving that Citrullin is a novel therapeutic agent in conditions of Arginine and NO deficiencies. Nitric Oxide (NO) plays pivotal roles in many physiological functions which are compromised in PCOS. The high levels of androgen and advanced glycation end products (AGEs) in PCOS women induces oxidative stress and inflammation. Recent reports show strong involvement of oxidative stress and endothelial dysfunction in PCOS pathogenesis. Both endothelial dysfunction and oxidative stress are prognostic indicators of cardio vascular disorders, pre-eclampsia, placenta-related defects and recurrent pregnancy loss. Endothelial function is compromised under PCOS, endothelial impairment being majorly attributed to endothelial nitric oxide (NO) synthesis, its bioavailability and oxidant-antioxidant mechanisms.

Specifically, the composition described herein comprises about 300, 350, 400, 450, 475, 500, 525, 550, 575, 600, 625, 650, 675, 700, 725 or 750 mg L-citrullin. L-citrullin may be provided by any suitable source. For example, L-citrullin can be purchased commercially, or obtained from chemical synthesis.

Berberine, berberine derivatives or analogs, salts thereof or mixtures thereof may be found in a variety plants, for example *Coptis chinensis* rhizomes (huanglian, coptis, goldthread), goldenseal, goldthread, *Phellodendron amurense* bark, *Berberis sargentiana, Berberis thunbergii, Berberis vulgaris* (Barberry), *Berberis aquifolium* (Oregon grape), *Hydrastis canadensis* (goldenseal), and *Berberis aristate* (tree turmeric).

Products from these and other berberine-containing herbal sources, including any preparation or extract therefrom, are contemplated as useful compositions comprising berberine (or berberine analogs, related compounds and/or derivatives) for use within the composition described herein. According to a preferred embodiment, berberine is present in the composition described herein as an extract of *Berberis aristata.*

Berberine, berberine derivatives or analogs, and salts thereof may be provided by any suitable source. For example, berberine can be purchased commercially, provided as plant extract from e.g. *Berberis aristata,* or obtained from chemical synthesis.

Berberine, berberine derivatives or analogs, and salts thereof may be prepared synthetically or semi-synthetically by a variety of chemical and/or enzymatic methods known in the art, for example, as described in US 2006/0223838 A1.

In one embodiment, the composition described herein comprises berberine, one or more pharmacologically acceptable salts of berberine or a mixture thereof. Preferred pharmacologically acceptable salts of berberine include, for example, acid addition salts, e.g. chlorides, sulfates, carbonates, phosphates, citrates and acetates. Acid addition salts may be produced by reacting berberine with an appropriate acid.

Monacolins are a group of molecules produced by fermentation of rice by the yeast *Monascus purpureus.* Fermentation of the species *Monascus purpureus* has been known for centuries in plant therapy and nutrition in China. The yeast is added to the rice (*Oryza sativa*) to allow its fermentation and the obtained product, known as red rice, is used in the food sector.

Monacolins have a structure similar to that of statins. Monacolin K has the same structure as lovastatin. Specifically, the composition described herein comprises about 20, 21, 22, 23, 24, 25, 26, 27, 28, 29, 30, 31, 32, 33, 34, 35, 36, 37, 38, 39, 40, 41, 42, 43, 45, 46, 47, 48, 49 or 50 mg monacolin K.

Monacolin K may be provided by any suitable source. For example, monacolin K can be purchased commercially, provided as extract of red yeast rice, or obtained from chemical synthesis.

The terms "red yeast rice", "red rice yeast", "red rice", "product of red rice", "extract of red rice" and the like refer to a product resulting from the fermentation performed by at least one fungus of the species *Monascus.* This fermentation can also be carried out by a mixture of fungi of the species *Monascus.*

Fermented red rice can be obtained through known methods, for example as described in EP1044009B1 (Detailed description of the invention; Preparation of conventional culture fluid, pages 5 to 6). To obtain the red rice, one or more natural or mutating strains of fungi of the species *Monascus* can be used, such as those comprised in the following list: *Monascus ruber van Tieghem, Monascus paxii Lingelsheim, Monascus purpureus, Monascus fuliginosus* and *Monascus albidus.*

According to a specific embodiment, monacolin K is included in the composition described herein as red yeast rice extract. Specifically, the red yeast rice extract comprised in the composition described herein contains an amount of monacolin K comprised between 0.001 % and 100 %, preferably between 0.001 % and 50 %, more preferably between 0.001 % and 25 %, and even more preferably between 0.001 % and 15 % by weight of the extract. According to a preferred embodiment of the composition provided herein, the red yeast rice extract comprised in said composition contains an amount of monacolin K between 10 % and 15 % by weight of the extract. Specifically, the red yeast rice extract as mentioned above comprises an amount of monacolin K of 0.001 %, 0.005 %, 0.01 %, 0.05 %, 0.1 %, 0.5 %, 1 %, 2 %, 3 %, 4 %, 5 %, 6 %, 7 %, 8 %, 9 %, 10 %, 11 %, 12 %, 13 %, 14 %, or 15 % by weight of the dry extract.

Specifically, the composition described herein comprises about 150, 155, 160, 165, 170, 175, 180, 185, 190, 195, 200, 205, 210, 215, 220, 225, 230, 235, 240, 245 or 250 mg red yeast rice extract.

As used herein, "policosanol" is a term used in its broadest sense and may refer to policosanol in many different chemical forms including policosanol and policosanol derivates. The policosanol may be one of or a mixture of the straight chain primary aliphatic alcohols (saturated and/or unsaturated) from about 20 to about 36 carbons in length. Policosanol may be derived from any particular plant or synthetic source, including sugar cane, rice wax, beeswax, yams, synthesized 1-octacosanol, and other sources. Forms of policosanol used include, by non-limiting example: 1-eicosanol (C-20), 1-docosanol (C-22), 1-tetracosanol (C-24), 1-hexacosanol (C-26), 1-heptacosanol (C-27), 1-octacosanol (C-28), 1-nonacosanol (C-29), 1-triacontanol (C-30), 1-dotriacontanol (C-32), 1-tetratriacontanol (C-34), and 1-hexatriacontanol (C-36), mixtures of the foregoing, derivatives of the foregoing, and/or mixtures of the foregoing and their derivatives.

Policosanol may be provided by any suitable source. For example, policosanol can be purchased commercially, or obtained from chemical synthesis.

Specifically, the composition described herein comprises about 5, 6, 7, 8, 9, 10, 11, 12, 13, 14, 15, 16, 17, 18, 19, 20, 21, 22, 23, 24, 25, 26, 27, 28, 29 or 30 mg policosanol, preferably between 10 to 15 mg, specifically about 10, 10.5, 11, 11.5, 12, 12.5, 13, 13.5, 14, 14.5 or 15 mg.

The composition described herein, and comprising either Mixture A or Mixture B as described herein, may be administered in a formulation comprising only said composition or may be administered in combination with further compounds or compositions, specifically a composition also referred to herein as the "Mixture C".

The composition provided herein, may be combined with further mixtures or compositions and administered simultaneously in a formulation combined with said further mixtures or compositions or sequentially in separate formulations.

The composition described herein may be administered together with the Mixture C in a single formulation or as separate formulations, e.g. in two separate dosage units formulated as powder or tablets.

### Mixture C

According to a specific embodiment, Mixture C comprises any one or more, preferably all of, thiamine mononitrate (vitamin B1), Riboflavin 5'-monophosphate sodium salt (vitamin B2), pyridoxal 5'-phosphate monohydrate (vitamin B6), hydroxocobalamin acetate (vitamin B12), choline bitartrate (vitamin B4), D-alpha-tocopherylacetate (vitamin E), selenomethionine, zinc orotate, manganese-(II)-gluconate-dihydrate, copper bisglycinate, potassium iodide and L-5-methyltetrahydrofolate. According to a further specific embodiment, the composition described herein further optionally comprises folate or folic acid, specifically L-5-methyltetrahydrofolate, and potassium iodide.

### Mixture D

According to a specific embodiment, Mixture D comprises pomegranate extract and the active probiotics *L. crispatus, L. gasseri, L. jensenii, L. reuteri* and *L. rhamnosus.* According to a specific example, but not limited thereto, Mixture D comprises 20% *L. crispatus,* 20% *L. gasseri, 20% L. jensenii, 20% L. reuteri,* and 20% *L. rhamnosus.*

Pomegranate, *Punica granatum L,* is a plant that grows naturally in Southwest Asia, India's northwest province and California, U.S.A, and is now widely cultivated in subtropical and tropical regions. Pomegranate used in the present disclosure is not limited to a particular type, but red pomegranate is preferred. Specific examples of red pomegranate include those from Iran, California, Taiwan, Uzbekistan, Turkey and Korea. The pomegranate extract may vary depending on production areas and harvest times of pomegranates used. An extract using pomegranate pulps alone without containing pomegranate pericarps and seeds is preferred. The pomegranate pericarps and seeds may cause adverse effects, for example, an allergic reaction such as tongue swelling may appear in some people after intake.

The pomegranate extract included in the compositions described herein may be prepared by the following method. For example, first, after cleaning pomegranates, pericarps and seeds are completely removed from the pomegranates, and enzyme such as pectinase, proteinase, amylase, and cellulase is added to degrade polysaccharides, such as starch, present in the pomegranates. Subsequently, optionally, an additive such as gelatin, silicon dioxide, bentonite, silicasol, tannin, cellulose, and potassium caseinate is added to control the turbidity, colour, and viscosity of the pomegranate extract, followed by concentration by heating, to produce a pomegranate extract, but the present disclosure is not limited thereto.

Specifically, Mixture D as described herein comprises at least 100, 200, 300, 400, 500, or 600 mg pomegranate extract. Preferably, Mixture D comprises at least about 150, 160, 170, 180, 190, 200, 210, 220, 230, 240 or 250 mg pomegranate extract.

The composition described herein may be administered together with Mixture C in a single formulation, e.g. formulated as a powder or a tablet, or as separate formulations, e.g. in separate dosage units formulated as powder or tablets.

Specifically, Mixtures D as described herein is contemplated as synbiotics. Synbiotics are formulations typically comprising probiotic bacteria and at least one prebiotic.

As used herein, "prebiotic" means a non-digestible food ingredient that beneficially affects the host by selectively stimulating the growth and/or activity of one or a limited number of bacteria in the colon and thus improves host health. (Gibson and Roberfroid, 1995 "Dietary Modulation of the Human Colonic Microbiota: Introducing the Concept of Prebiotics" J. Nutr 125:1401-1412). For example, prebiotics may be any one or more of inulin, pomegranate extract, fructooligosaccharide (FOS), galacto-oligosaccharides, lactulose, resistant starch, cellulose, hemicellulose, pectin and/or gum.

As used herein, "probiotic bacteria" means microbial cell preparations or components of microbial cells with a beneficial effect on the health or well-being of the host. (Salminen S, et al "Probiotics: how should they be defined" Trend Food Sci. Technol. 1999:10 107-10).

Specifically the composition comprises 8 to 15 mg of *L. crispatus* (50bn cfu/g), preferably 12mg, 8 to 15 mg of *L. gasseri* (50bn cfu/g), preferably 12mg, 8 to 15 mg of *L. jensenii* (50bn cfu/g), preferably 12mg, 8 to 15 mg of *L. reuteri* (50bn cfu/g), preferably 12mg, and 8 to 15 mg of *L. rhamnosus* (50bn cfu/g), preferably 12mg.

According to a further specific embodiment, the composition described herein, optionally comprising Mixture C, Mixture D, further optionally comprises Mixture E or is administered together with Mixture E.

### Mixture E

According to a specific embodiment, the composition provided herein further comprises an optional mixture ("Mixture E") comprising any one or more Vitex agnus-castus, Black cohosh extract, and/or 5-L-Hydroxytryptophan. According to a further specific embodiment, Mixture E comprises any one or more 8-16mg Vitex agnus-castus, preferably 12mg; 4-10 mg Black cohosh extract, preferably about 7 mg; and/or 50 to 200 mg 5-L-Hydroxytryptophan, preferably about 100 mg.

Specifically, Vitex agnus-castus is added to the composition as the pure chemical compound, e.g. synthetically produced agnuside, or it is included in the composition as plant extract, preferably as an extract of *Vitex agnus-castus.*

Specifically, Black cohosh extract is added to the composition as the pure chemical compound, *e.g.* synthetically produced triterpene glycosides, or are included in the composition as plant extract, preferably as an extract of *Cimicifuga racemosa.*

Specifically, 5-L-Hydroxytryptophan is added to the composition as the pure chemical compound, *e.g.* synthetically produced, or is included in the composition as plant extract, preferably as an extract of *Griffonia simplicifolia.*

The composition described herein, may be administered together with Mixture C and or Mixture D in a single formulation, e.g. formulated as a powder or a tablet, or as separate formulations, e.g. in separate dosage units formulated as powder or tablets.

In one embodiment, the compositions described herein are in the form of a dietary supplement. As used herein the term "dietary supplement" refers to a composition to supplement the diet in a physical form for ingestion, such as a pill, capsule, tablet, powder or liquid form that is not a drug. In certain embodiments, this disclosure relates to dietary supplements comprising the formulation or composition disclosed herein optionally in combination with other ingredients of a dietary supplement such as vitamins, minerals, herbs, concentrates, or metabolites. Specifically, the dietary supplement comprises an effective amount of the composition as defined above, optionally together with one or more edible excipients or vehicles.

In one embodiment, optionally combined with any other embodiment of the invention described herein above or below, the composition of the present invention is in the form of a pharmaceutical composition. The pharmaceutical composition comprises a therapeutically effective amount of the composition, as defined above, together with one or more pharmaceutically acceptable excipients or vehicles. The excipients or vehicles which are used are for oral administration, including but not limited to fillers, binders, disintegrants, lubricants, anti-caking agents, glidants or mixtures thereof.

The pharmaceutical compositions and dietary supplements of the invention can be formulated in numerous forms which include, but are not limited to, capsules, powder, water-soluble powder, granules, suspensions, powders, chewable tablets, gums, gelatins, oils, chewable gelatin forms, or chewable tablets.

Methods for preparing dietary supplements and/or pharmaceutical compositions are well known in the state of the art. Those skilled in the art can readily determine the suitable excipients and their amounts according to the type of formulation that is being prepared.

Excipients are the constituents of a pharmaceutical form apart from the active substance. Excipients include e.g. fillers, disintegrants, lubricants, colouring matters, antioxidants, preservatives, adjuvants, stabilisers, thickeners, emulsifiers, solubilisers, permeation enhancers, flavouring and aromatic substances etc., as well as the constituents of the outer covering of the medicinal products, e.g. gelatine capsules. Excipients will be used as described in the European Pharmacopoeia.

A preparation described herein is specifically administered at an effective amount, in particular a therapeutically effective amount, meaning a quantity or activity sufficient to effect beneficial or desired results, including clinical results, when administered to a subject, e.g. a patient suffering from a disease or disorder as described herein. As such, an effective amount or synonymous quantity thereof depends upon the context in which it is being applied. An effective amount is intended to mean that amount of a compound that is sufficient to treat, prevent or inhibit such diseases or disorders.

Specifically, the preparation described herein may be administered once or twice a day. Specifically, the preparation described herein is administered at least any one of 1, 2, 3, 4, or 5 times a day, or more.

The amount of the preparation described herein that will correspond to such an effective amount will vary depending on various factors, such as the given drug or active substances, the pharmaceutical formulation, the route of administration, the type of disease or disorder, the identity of the subject or host being treated, and the like, but can nevertheless be routinely determined by one skilled in the art.

The dietary supplements or pharmaceutical preparations provided herein may be used to treat a disease or disorder such as polycystic ovary syndrome (PCOS), and diabetes-related disorders such as e.g. insulin-resistance, obesity, dyslipidemia, Type 2 diabetes, gestational diabetes, hyperandrogenemia, hirsutism, decreased FSFI, decreased sexual function and libido, difficulties with conception and metabolic syndrome. The dietary supplements or pharmaceutical preparations provided herein may also be used to improve symptoms associated with menopause or premenstrual syndrome (PMS), or to improve difficulties with ovulation, e.g. by increasing ovulation rate.

As used herein, the term "treatment" is meant to include both prophylactic and therapeutic treatment.

The prevalence of metabolic disturbance, collectively known as metabolic syndrome or insulin resistance syndrome, has reached an epidemic proportion in industrialized countries. The insulin resistance syndrome refers to a constellation of findings, including glucose intolerance, obesity, an altered lipid profile (dyslipidemia) and hypertension, that promote the development of type 2 diabetes, cardiovascular disease, cancer, polycystic ovarian disease, decreased FSFI and other disorders. In all of these disorders, a central component of the pathophysiology is insulin resistance.

"Diabetes-related disorders" include for example Type 2 Diabetes, maturity onset diabetes of the young (MODY), impaired fasting glucose (IFG), impaired glucose tolerance (IGT), insulin resistance, elevated LDL levels, hyperandrogenemia and elevated testosterone levels, and metabolic syndrome.

Insulin is a hormone that is produced by the beta cells of the pancreas. The insulin produced is released into the blood stream and is transported throughout the body. Insulin is an important hormone that has many actions within the body. Most of the actions of insulin are directed at metabolism (control) of carbohydrates (sugars and starches), lipids (fats), and proteins. Insulin also is important in regulating the cells of the body including their growth.

The term "insulin resistance" or "IR" in short, is used to refer to a condition in which the cells of the body become resistant to the effects of insulin, that is, the normal response to a given amount of insulin is reduced. As a result, higher levels of insulin are needed in order for insulin to have its effects. Insulin resistance precedes the development of type 2 diabetes, sometimes by several years. In individuals who will ultimately develop type 2 diabetes, it is believed that blood glucose and insulin levels are normal for many years; then at some point in time, insulin resistance develops. Accordingly, the treatment of the cause of insulin resistance is preferred over treatment of the symptoms of diabetes. The present invention is primarily aimed at providing a medicament for use in the treatment of insulin resistance.

"Type 2 diabetes" or "T2D" (OMIM 125853), in some embodiments, is defined as provided by the World Health Organization and the International Diabetes Federation in "Definition and diagnosis of diabetes mellitus and intermediate hyperglycemia", published in 2006. In more particular embodiments, a diabetic subject exhibits a fasting plasma glucose of >=126 mg/dL or a 2-hour plasma glucose (2 hours after oral administration of 75 grams of glucose) >=200 mg/dL. In some embodiments a diabetic or pre-diabetic subject exhibits elevated levels of glycated hemoglobin, e.g., greater than 4.5, 4.6, 4.7, 4.8, 4.9, 5.0, 5.1, 5.2, 5.3, 5.4, 5.5, 5.6, 5.8, 6.0, 6.2, 6.4, 6.6., 6.8, 7.0, 7.2, 7.4, 7.6%, or more of total hemoglobin.

The term "metabolic syndrome" refers to a group of symptoms that occur together and increase the risk for coronary artery disease, stroke and type 2 diabetes. In some embodiments the subject has central obesity (waist circumference>=80 cm for women; >=90 cm for Asian men, including ethnic South and Central Americans, and >=94 cm for all other males), BMI>30 kg/m², raised triglycerides (>=150 mg/dL, or specific treatment for this lipid abnormality), reduced HDL cholesterol (<40 mg/dL in males, <50 mg/dL in females or specific treatment for this lipid abnormality), raised blood pressure (sBP>=130 mm HG or dBP>=85 mm HG or treatment of previously diagnosed hypertension) or raised fasting plasma glucose (FPG>=100 mg/dL or previous type 2 diabetes diagnosis), including combinations thereof. In more particular embodiments, the subject to be treated by the methods provided by the invention has or is at increased risk for metabolic syndrome, as defined by the International Diabetes Federation in "The IDF consensus worldwide definition of the metabolic syndrome," published in 2006, i.e., the subject has central obesity (as described above, and/or BMI>30 kg/m²) and any two of raised triglycerides, reduced HDL cholesterol, raised blood pressure, or raised fasting plasma glucose.

"Polycystic ovarian syndrome" or "polycystic ovary syndrome" or "PCOS" is characterized by one or more of bilateral enlarged ovaries, abnormal 24-hour urinary ketosteroids, and evidence of virilization and, in particular embodiments, all three indications. In certain particular embodiments, PCOS is correlated with one or more of obesity, hirsutism, and amenorrhea, e.g., 1, 2, or all 3 indications. In other embodiments, a subject with PCOS exhibits elevated urinary levels of PSA (human GenelD No. 354) and/or kallikrein-2 (human GenelD No. 3817). In certain particular embodiments, PCOS is characterized by the presence of a mutation in any one of the loci identified in OMIM accession number 184700, including follistatin, CYP11A, CAPN10, or INSR (human GenelD No. 3643). Diagnosis of PCOS by Rotterdam criteria requires two of three criteria in women, including oligo- and/or anovulation, clinical or biochemical hyperandrogenism and polycystic ovaries by ultrasound.

In a particular embodiment, the invention relates to a pharmaceutical preparation or dietary supplement consisting of a number of separately packaged and individually removable daily dose units placed in a packaging unit and intended for oral administration for a period of at least 18, 19, 20, 21, 22, 23, 24, 25, 26, 27, 28, 29, 30, or 31 consecutive days, wherein each of said daily dosage units comprises a composition comprising or consisting of either Mixture A or Mixture B as described herein.

In a further particular embodiment, certain daily dose units comprise the composition consisting of L-citrullin and silymarin and either Mixture A or Mixture B as described herein and in addition comprise Mixture C and/or Mixture D and/or Mixture E as described herein.

It is thus contemplated that the dietary supplement or pharmaceutical preparation described herein is a multiphase product, which is administered over a period of at least 28 consecutive days comprising different daily dose units, depending on the day in the cycle of at least 28 consecutive days. As described above, the multiphase product may comprise two different compositions, one administered for the full cycle, the second administered during follicular phase, preferably from day 8 to day 14 of the menstrual cycle.

Preferably, the composition comprising either Mixture A or Mixture B, and Mixture C as described above, is administered each day of the at least 28-day cycle in a single formulation, e.g. as water-soluble powder or as a capsule. Alternatively, the composition and/or Mixture D may be administered in separate formulations, e.g. one as powder and the other as capsule, or both as separate powders or capsules.

The effectiveness of a composition described herein for treating or preventing (i.e., reducing or decreasing the likelihood of developing or occurrence of) a metabolic disease (e.g., insulin-resistance) or a fertility disorder, such as for example a decreased ovulation rate, can readily be determined by a person skilled in the medical and clinical arts. One or any combination of diagnostic methods, including physical examination, assessment and monitoring of clinical symptoms, and performance of analytical tests and methods described herein and practiced in the art may be used for monitoring the health status of the subject. The effects of the treatment using the compositions described herein can be analyzed using techniques known in the art, such as comparing symptoms of patients suffering from or being at risk of metabolic disease or fertility problems that have received the treatment with those of patients without such a treatment or with placebo treatment.

Specifically, to determine a treatment effect in women suffering from PCOS or decreased fertility, the level of Testosterone, DHEAS; SHGB and/or LH may be determined, as well as ovulation rate and menstrual cycle status. Specifically, a decrease in the levels of testosterone, DHEAS and/or LH is indicative of treatment success in female patients suffering from PCOS or decreased fertility. Specifically, an increase in the ovulation rate, e.g. determined via ultrasound, is indicative of treatment success in female patients suffering from PCOS or decreased fertility. Specifically, an improvement in any one or more of IFG (Impaired fasting glucose), IGT (Impaired glucose tolerance) and LDL levels; FSFI, HOMA1-IR, TSH, PRL, and 17-hydroxyprogesterone, SHBG, prolactin, testosterone and dehydroepiandrosterone sulphate are indicative of treatment success in female patients suffering from PCOS or decreased fertility.

The invention is further embodied by the following items:
1. A composition comprising L-citrullin and silymarin; and
   a. Mixture A comprising:
      i. myo-inositol;
      ii. D-chiro-inositol;
      or
   b. Mixture B comprising:
      i. berberine;
      ii. monacolin K; and
      iii. policosanol.
2. The composition of item 1, wherein the composition comprising Mixture A comprises 150 to 180mg Silymarin, 300 to 750mg L-citrullin, 3.900 to 4.100 mg myo-inositol, and 30 to 70 mg D-chiro-inositol.
3. The composition of item 1, wherein the composition comprising Mixture B comprises 150 to 180mg Silymarin, 300 to 750mg L-citrullin, 400 to 600 mg berberine, 10 to 30 mg of a synthetic monacolin K compound or 200 to 250 mg red yeast rice extract, and 10 to 20 mg policosanol.
4. The composition of any one of items 1 to 3, further comprising any one or more of thiamine mononitrate (vitamin B1), Riboflavin 5'-monophosphate sodium salt (vitamin B2), pyridoxal 5'-phosphate monohydrate (vitamin B6), hydroxocobalamin acetate (vitamin B12), choline bitartrate (vitamin B4), D-alpha-tocopherylacetate (vitamin E), selenomethionine, zinc orotate, manganese-(II)-gluconate-dihydrate, copper bisglycinate, potassium iodide and L-5-methyltetrahydrofolate.
5. The composition of any one of items 1 to 4, further comprising 5-L-Hydroxytryptophan, and/or Vitex agnus-castus extract or agnusides, and/or Black cohosh extract or triterpene glycosides.
6. The composition of item 5, comprising 50 to 200 mg 5-L-Hydroxytryptophan, preferably about 100 mg, and/or 8-16mg Vitex agnus-castus, preferably 12mg, and/or and 4-10 mg Black cohosh extract, preferably about 7 mg.
7. The composition of any one of items 1 to 6, further comprising the bacterial strains *L. crispatus, L. gasseri, L. jensenii, L. reuteri* and *L. rhamnosus.*
8. The composition of item 7, wherein the mixture of bacterial strains further comprises 100 mg to 300 mg pomegranate extract, preferably 200 mg.
9. The composition of any one of items 1 to 8, further comprising one or more pharmaceutically acceptable carriers.
10. The composition of any one of items 1 to 9, wherein the composition is formulated in the form of a powder, preferably a water-soluble powder or a capsule.
11. The composition of any one of items 1 to 10, for use in the treatment of a disease or disorder.
12. The composition for use according to item 11, wherein the disease or disorder is selected from the group consisting of polycystic ovary syndrome (PCOS), difficulties with ovulation, decreased female sexual function index (FSFI), menopause, premenstrual syndrome (PMS), insulin-resistance, obesity, dyslipidemia, coronary heart disease, metabolic syndrome, weight gain, and increased appetite, reduced sexual desire, and hyperandrogenemia (HA).
13. A dietary supplement comprising the composition of any one of items 1 to 10.
14. The dietary supplement of item 13, which is in the form of a powder, preferably a water-soluble powder, a tablet, a capsule, or a solid or semi-solid candy, or a combination of one or more dosage forms.
15. Use of the dietary supplement of item 13 or 14, to reduce insulin-resistance, to increase the rate of ovulation, or to decrease hyperandrogenism, balance the menstrual cycle and hormones (prolactin, testosterone and dehydroepiandrosterone sulphate), or to improve female sexual function (FSFI).
16. Use of the dietary supplement of item 13 or 14, to reduce menopause-associated symptoms such as insulin resistance, polyphagia, vaginal dryness, weight gain, reduced sexual desire, and/or hormonal-related mood swings.
17. Use of the dietary supplement of item 13 or 14, to improve metabolic syndrome and hyperandrogenemia in female subjects.
18. A pharmaceutical preparation consisting of a number of separately packaged and individually removable daily dose units placed in a packaging unit and intended for oral administration for a period of at least 21 consecutive days, preferably at least 28 consecutive days, wherein said daily dose units comprise a composition comprising L-citrullin and silymarin and
   a. Mixture A comprising:
      i. myo-inositol;
      ii. D-chiro-inositol;
      or
   b. Mixture B comprising:
      i. berberine;
      ii. monacolin K; and
      iii. policosanol.
19. A multiphase product for enhanced female fertility, comprising at least 28 consecutive daily dose units of a composition comprising L-citrullin and silymarin and
   a. Mixture A comprising:
      i. myo-inositol;
      ii. D-chiro-inositol;
      or
   b. Mixture B comprising:
      i. berberine;
      ii. monacolin K; and
      iii. policosanol; and wherein
   the full-cycle additionally comprises Mixture C, which is administered for 28 consecutive days and Mixture D, which is additionally administered during follicular phase for 6 consecutive days, specifically from day 8 to 13.
20. The multiphase product of item 19, wherein the basic composition further comprises thiamine mononitrate (vitamin B1), Riboflavin 5'-monophosphate sodium salt (vitamin B2), pyridoxal 5'-phosphate monohydrate (vitamin B6), hydroxocobalamin acetate (vitamin B12), choline bitartrate (vitamin B4), D-alpha-tocopherylacetate (vitamin E), selenomethionine, zinc orotate, manganese-(II)-gluconate-dihydrate, copper bisglycinate, potassium iodide and L-5-methyltetrahydrofolate.
21.The multiphase product of item 19 or 20, wherein Mixture D is further administered from day 8 to day 13, and comprises pomegranate extract and the bacterial strains *L. crispatus, L. gasseri, L. jensenii, L. reuteri* and *L. rhamnosus.*
22. A method of treating a disease, comprising the steps of administering to a patient in need there of a composition comprising L-citrullin and silymarin and
   a. Mixture A comprising:
      i. myo-inositol;
      ii. D-chiro-inositol;
      or
   b. Mixture B comprising:
      i. berberine;
      ii. red yeast rice; and
      iii. policosanol.
23. The method of item 22, wherein the composition is formulated in an oral formulation using a pharmaceutically acceptable carrier.
24. The method of item 23, wherein the oral formulation is in the form of a water-soluble powder, or a capsule.

### EXAMPLES

The examples described herein are illustrative of the present invention and are not intended to be limitations thereon. Many modifications and variations may be made to the techniques described and illustrated herein without departing from the spirit and scope of the invention. Accordingly, it should be understood that the examples are illustrative only and are not limiting upon the scope of the invention.

| **Mixture A** | |
|---|---|
| **Name of ingredients** | **Additional information about ingredients** |
| Myo-Inositol | CAS 142583-61-7 |
| D-Chiro Inositol | CAS 33889-69-9 |
| Silymarin phyproof^{®} | CAS 33889-69-9 |
| L-Citrullin | CAS 372-75-8 |

| **Mixture B** | |
|---|---|
| **Name of ingredients** | **Additional information about ingredients** |
| Berberinchloride | CAS 633-65-8 |
| Monacolin K phyproof^{®} | CAS 75330-75-5 |
| Policosanol | CAS 142583-61-7 |
| Silymarin phyproof^{®} | CAS 33889-69-9 |
| L-Citrullin | CAS 372-75-8 |

| **Mixture C** | |
|---|---|
| **Name of ingredients** | **Additional information about ingredients** |
| Thiaminmononitrat | CAS 532-43-4 |
| Riboflavin 5'-monophosphate sodium salt | CAS 532-43-4 |
| Pyridoxal 5'-phosphate monohydrate | CAS 41468-25-1 |
| Hydroxocobalamin acetat | CAS 22465-48-1 |
| Choline bitartrate | CAS 87-67-2 |
| Selenomethionine | CAS 3211-76-5 |
| Zincorotate | CAS 60388-02-5 |
| Managanese-(II)-Gluconate-Dihydrat | CAS 6485-39-8 |
| Copper Bisglycinate | CAS 13479-54-4 |
| D-Alpha-Tocopheryl Acetate | CAS 7695-91-2 |
| L-5-methyltetrahydrofolate Magnafolate^{®} | CAS 26560-38-3 |
| Potassium iodide | CAS 7681-11-0 |

| **Mixture E** | |
|---|---|
| **Name of ingredients** | **Additional information about ingredients** |
| Powdered Black Cohosh Extract | CAS 84776-26-1 |
| Agnus Castus dry Fruit Extract | CAS 479-91-4 |
| 5-L-Hydroxytryptophan phyproof^{®} | CAS 4350-09-8 |

| **Mixture D** | |
|---|---|
| **Name of ingredients** | **Additional information about ingredients** |
| Lactobacillus crispatus matrix protected 50 Mrd cfu/g | |
| Lactobacillus gasseri matrix protected 50 Mrd cfu/g | |
| Lactobacillus jensenii matrix protected 50 Mrd cfu/g | |
| Lactobacillus reuteri matrix protected 50 Mrd cfu/g | |
| Lactobacillus rhamnosus matrix protected 50 Mrd cfu/g | |
| Punica granatum extract | CAS 84961-57-9 |

### EXAMPLE 1: Use of Mixture B in comparison to a combination of mixtures B+C+E in the treatment of PCOS, decreased FSFI, and insulin-resistance in premenopausal women

The following mixtures were used in this Example:

**Table 1: Mixture B**

| **Name of ingredients** | **Additional information about ingredients** | **Quantity (mg)** |
|---|---|---|
| Berberinchloride | CAS 633-65-8 | 500 |
| Monacolin K phyproof^{®} | CAS 75330-75-5 | 30 |
| Policosanol | CAS 142583-61-7 | 12 |
| Silymarin phyproof^{®} | CAS 33889-69-9 | 120 |
| L-Citrullin | CAS 372-75-8 | 500 |

**Table 2: Combination of Mixture B+C+E**

| **Name of ingredients** | **Additional information about ingredients** | **Quantity (mg)** |
|---|---|---|
| Berberinchloride | CAS 633-65-8 | 500 |
| Monacolin K phyproof^{®} | CAS 75330-75-5 | 30 |
| Policosanol | CAS 142583-61-7 | 12 |
| Silymarin phyproof^{®} | CAS 33889-69-9 | 120 |
| L-Citrullin | CAS 372-75-8 | 500 |
| Agnus Castus dry Fruit Extract** | CAS 479-91-4 | 10 |
| thiamine mononitrate* | CAS 532-43-4 | 1,1 |
| Riboflavin 5'-monophosphate sodium salt* | CAS 532-43-4 | 1,05 |
| Pyridoxal 5'-phosphate monohydrate* | CAS 41468-25-1 | 1,05 |
| Hydroxocobalamin acetat* | CAS 22465-48-1 | 0,0025 |
| Choline bitartrate* | CAS 87-67-2 | 82,5 |
| Selenomethionine* | CAS 3211-76-5 | 0,04125 |
| Zincorotate* | CAS 60388-02-5 | 7,5 |
| Managanese-(II)-Gluconate-Dihydrat* | CAS 6485-39- 8 | 1,5 |
| Copper Bisglycinate* | CAS 13479-54-4 | 0,75 |
| D-Alpha-Tocopheryl Acetate* | CAS 7695-91-2 | 9 |
| * Mixture C | | |
| ** Mixture E | | |

### Treatment

Study subjects receive oral doses of the compositions described above. Mixture B will be administered in two capsules. Mixtures B, C and E will be administered in two capsules.

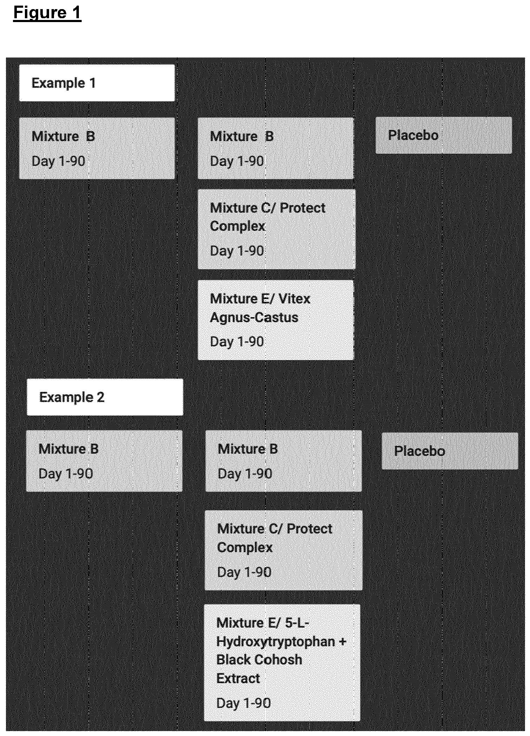

As control, a group of study subjects receive oral placebos. The treatment regimen is depicted in Fig. 1.

### Treatment Duration

### 3 months

### Study Design

Four women with regular menses (every 28-30 d) fulfilling below criteria are recruited for each research question. The mean body mass index (BMI) in the study population is comparable as are age, weight and ethnicity, following (Dunaif A, et al. Profound peripheral insulin resistance, independent of obesity, in polycystic ovary syndrome. Diabetes. 1989; 38:1165-74. [PubMed: 2670645]).

No subject should be taking any medication at the start of the study or for the preceding 6 months, and there should be no concurrent illness. All subjects are on an unrestricted diet and are instructed not to modify their usual eating patterns during the period of sampling.

### Insulin resistance

Insulin resistance is diagnosed using NIH criteria (1 Dunaif A., Insulin resistance and the polycystic ovary syndrome: mechanism and implications for pathogenesis. Endocr Rev. 1997; 18:774-800. [PubMed: 9408743]) and Rotterdam diagnostic criteria (Moran L, and Teede H. Metabolic features of the reproductive phenotypes of polycystic ovary syndrome. Hum Reprod Update. 2009; 15:477-88. [PubMed: 19279045]). Thus, the study includes women with the most marked metabolic abnormalities - hyperandrogenism and chronic anovulation (i.e. NIH criteria) - independent of polycystic ovaries.

### PCOS

In PCOS a low SHBG concentration and a low DHEAS level is an integrated marker of insulin resistance and may reflect insulin resistance more consistently than HOMA-IR itself. However, the low index of individuality of SHBG caused by the relatively higher inter- to intraindividual variation seen in PCOS suggests that it is unsuitable as a screening marker for insulin resistance alone, but, rather, that it is a marker for insulin resistance only after the diagnosis of PCOS. Once the diagnosis of PCOS is made, then a low SHBG concentration may be useful to identify those individuals with PCOS who are insulin resistant for targeted treatment with insulin-sensitizing agents like Mixture A or mixture B.

### Data collection

Laboratory assays are performed at the beginning and at the end of the study. Venous blood samples are collected from the antecubital vein between 8 and 9 am after an overnight 12-h fast. Moreover, glucose levels are measured in samples obtained 2 h after the oral ingestion of 75 g glucose. Plasma levels of glucose and serum levels of insulin are assayed by routine laboratory techniques using commercially available kits.

The homeostatic model for insulin resistance (HOMA1-IR) is calculated as follows: fasting glucose (mg/dL) × fasting insulin (mIU/L)/405.

Shortly after blood collection, all women complete a questionnaire assessing demographic characteristics, smoking, physical activity, education, occupation, stress exposure, general health, number and duration of marriages, number of sexual partners, deliveries and abortions, as well as questionnaires measuring female sexual function and depressive symptoms.

LH-Levels and ovulation will be assessed during mid-cycle using the at-home-ovulation predictor kit "Clearplan Easy Fertility Monitor (CPEFM)", assessing urinary hormone (LH) and estrone-3-glucuronide (E3G).

Endometrial thickness will be assessed using a transvaginal transducer in days 6-10 of the menstrual cycle. The endometrial thickness is considered as the widest distance from the reflective interface between the endometrium and the myometrium of opposite sides on a sagittal view of the uterus. BMI was related to endometrial thickness both in PCOS women without IR and in women with IR and without PCOS suggests that both IR and anovulation are factors influencing BMI's correlation to endometrial thickness.

The female sexual function index (FSFI) is a validated test evaluating all phases of the female sexual cycle, sexual satisfaction and dyspareunia in the last 4 weeks. It is composed of 19 items divided into 6 collective domains (subscales): sexual desire, sexual arousal, lubrication, orgasm, satisfaction and pain. The items are scored on a 5-point or 6-point scale, with responses ranging from 0 to 5 or 1 to 5 (0 indicates no sexual activity in the past month).

The presence and severity of depressive symptoms will be evaluated using the Beck Depression Inventory-Second Edition (BDI-II).

### Results

Mixture B, optionally in combination with Mixtures E and C, improves symptoms associated with insulin resistance and PCOS and specifically:
- reduces hirsutism and androgen levels, as documented with an absolute reduction in free testosterone,
- balances reproductive hormones as documented by changes in TSH, PRL, and 17-hydroxyprogesterone, SHBG, prolactin, testosterone and dehydroepiandrosterone sulphate,
- improves insulin parameters (LDL, HDL, triglycerides, IGT or IFT),
- changes in FSFI: correlations between HOMA1-IR and desire or sexual satisfaction should be stronger than correlations between HOMA1-IR and the remaining domains assessed by FSFI,
- decreases insulin resistance, as documented by HOMA1-IR,
- changes in endometrium thickness,
- decreases IGF-I,
- irrespective of the underlying condition, sexual functioning improves after insulin-sensitizing treatment.

### EXAMPLE 2: Use of Mixture B in comparison to a combination of mixtures B+C+E in the treatment of hyperandrogenism, decreased FSFI, subclinical depression and insulin-resistance in perimenopausal women

The following mixtures were used in this Example:

**Table 3: Mixture B**

| **Name of ingredients** | **Additional information about ingredients** | **Quantity (mg)** |
|---|---|---|
| Berberinchloride | CAS 633-65-8 | 500 |
| Monacolin K phyproof^{®} | CAS 75330-75-5 | 30 |
| Policosanol | CAS 142583-61-7 | 12 |
| Silymarin phyproof^{®} | CAS 33889-69-9 | 120 |
| L-Citrullin | CAS 372-75-8 | 500 |

**Table 4: Combination of Mixtures B+C+E**

| **Name of ingredients** | **Additional information about ingredients** | **Quantity (mg)** |
|---|---|---|
| Berberinchloride | CAS 633-65-8 | 500 |
| Monacolin K phyproof^{®} | CAS 75330-75-5 | 30 |
| Policosanol | CAS 142583-61-7 | 12 |
| Silymarin phyproof^{®} | CAS 33889-69-9 | 120 |
| L-Citrullin | CAS 372-75-8 | 500 |
| 5-L-Hydroxytryptophan phyproof^{®}** | CAS 4350-09-8 | 75 |
| Powdered Black Cohosh Extract** | CAS 84776-26-1 | 7 |
| Tthiamine mononitrate* | CAS 532-43-4 | 1,1 |
| Riboflavin 5'-monophosphate sodium salt* | CAS 532-43-4 | 1,05 |
| Pyridoxal 5'-phosphate monohydrate* | CAS 41468-25-1 | 1,05 |
| Hydroxocobalamin acetat* | CAS 22465-48-1 | 0,0025 |
| Choline bitartrate* | CAS 87-67-2 | 82,5 |
| Selenomethionine* | CAS 3211-76-5 | 0,04125 |
| Zincorotate* | CAS 60388-02-5 | 7,5 |
| Managanese-(II)-Gluconate-Dihydrat* | CAS 6485-39- 8 | 1,5 |
| Copper Bisglycinate* | CAS 13479-54-4 | 0,75 |
| D-Alpha-Tocopheryl Acetate* | CAS 7695-91-2 | 9 |
| ** Mixture C* | | |
| ** Mixture E | | |

### Treatment

- Mixture B in comparison to
- a combination of mixtures B, C and E
- and placebo

Study subjects receive oral doses of the compositions described above. Mixture B will be administered in two capsules.
A combination of mixtures B, C and E will be administered in two capsules.

As control, a group of study subjects receive oral placebos. The treatment regimen is depicted in Fig. 1.

### Treatment Duration

### 3 months

### Study Design

Four perimenopausal women complaining of decreased sexual desire for a period of at least 3 months will be included in the study. Perimenopausal women should not have many menses for a least six months and have elevated LH and FSH levels.

The mean body mass index (BMI) in the study population will be comparable as will be age, weight and ethnicity. No subject should be taking any medication at the start of the study or for the preceding 6 months, and there should be no concurrent illness. All subjects will be on an unrestricted diet and are instructed not to modify their usual eating patterns during the period of sampling.

Laboratory assays will be performed at the beginning and at the end of the study. Venous blood samples will be collected from the antecubital vein between 8 and 9 am after an overnight 12-h fast. Moreover, glucose levels will be measured in samples obtained 2 h after the oral ingestion of 75 g glucose. Plasma levels of glucose and serum levels of insulin will be assayed by routine laboratory techniques using commercially available kits.

The homeostatic model for insulin resistance (HOMA1-IR) will be calculated as follows: fasting glucose (mg/dL) × fasting insulin (mIU/L)/405.

Shortly after blood collection, all women will complete a questionnaire assessing demographic characteristics, smoking, physical activity, education, occupation, stress exposure, general health, number and duration of marriages, number of sexual partners, deliveries and abortions, as well as questionnaires measuring female sexual function and depressive symptoms.

The female sexual function index (FSFI) is a validated test evaluating all phases of the female sexual cycle, sexual satisfaction and dyspareunia in the last 4 weeks. It is composed of 19 items divided into 6 collective domains (subscales): sexual desire, sexual arousal, lubrication, orgasm, satisfaction and pain. The items are scored on a 5-point or 6-point scale, with responses ranging from 0 to 5 or 1 to 5 (0 indicates no sexual activity in the past month).

The presence and severity of depressive symptoms will be evaluated using the Beck Depression Inventory-Second Edition (BDI-II).

### Results

Mixture B, and Mixture B combination with Mixtures E and C, improve symptoms associated with insulin resistance and PCOS and specifically:
- reduce hirsutism and androgen levels, as documented with an absolute reduction in free testosterone,
- balance reproductive hormones as documented by changes in TSH, PRL, and 17-hydroxyprogesterone, SHBG, prolactin, testosterone and dehydroepiandrosterone sulphate,
- improve insulin parameters (LDL, HDL, triglycerides, IGT or IFT),
- induce changes in FSFI: correlations between HOMA1-IR and the domains assessed by FSFI,
- increase DHEAS,
- decrease insulin resistance, as documented by HOMA1-IR,
- Irrespective of the underlying condition, sexual functioning improves after insulin-sensitizing treatment.

### EXAMPLE 3: Use of Mixture A, in comparison to a combination of Mixtures B+C, in comparison to a combination of Mixtures A+C, in comparison to Mixtures B+C+D for improvement of ovulation, regulation of the menstrual cycle and balancing hormonal levels in women with clinical or sub-clinical PCOS trying to conceive

The following mixtures were used in this Example:

**Table 5: Mixture A**

| **Name of ingredients** | **Additional information about ingredients** | **Quantity (mg)** |
|---|---|---|
| Myo-Inositol | CAS 142583-61-7 | 12 |
| D-Chiro Inositol | CAS 33889-69-9 | 120 |
| Silymarin phyproof^{®} | CAS 33889-69-9 | 120 |
| L-Citrullin | CAS 372-75-8 | 500 |

**Table 6: Combination of Mixtures B+C**

| **Name of ingredients** | **Additional information about ingredients** | **Quantity (mg)** |
|---|---|---|
| Berberinchloride | CAS 633-65-8 | 500 |
| Monacolin K phyproof^{®} | CAS 75330-75-5 | 30 |
| Policosanol | CAS 142583-61-7 | 12 |
| Silymarin phyproof^{®} | CAS 33889-69-9 | 120 |
| thiamine mononitrate* | CAS 532-43-4 | 1,1 |
| Riboflavin 5'-monophosphate sodium salt* | CAS 532-43-4 | 1,05 |
| Pyridoxal 5'-phosphate monohydrate* | CAS 41468-25-1 | 1,05 |
| Hydroxocobalamin acetat* | CAS 22465-48-1 | 0,0025 |
| Choline bitartrate* | CAS 87-67-2 | 82,5 |
| Selenomethionine* | CAS 3211-76-5 | 0,04125 |
| Zincorotate* | CAS 60388-02-5 | 7,5 |
| Managanese-(II)-Gluconate-Dihydrat* | CAS 6485-39- 8 | 1,5 |
| Copper Bisglycinate* | CAS 13479-54-4 | 0,75 |
| D-Alpha-Tocopheryl Acetate* | CAS 7695-91-2 | 9 |
| L-5-methyltetrahydrofolate Magnafolate^{®} * | CAS 26560-38-3 | 0,7 |
| Potassium iodide * | CAS 7681-11-0 | 0,75 |
| **Mixture C* | | |

**Table 7: Combination of Mixtures A+C**

| **Name of ingredients** | **Additional information about ingredients** | **Quantity (mg)** |
|---|---|---|
| Myo-Inositol | CAS 142583-61-7 | 12 |
| D-Chiro Inositol | CAS 33889-69-9 | 120 |
| Silymarin phyproof^{®} | CAS 33889-69-9 | 120 |
| L-Citrullin | CAS 372-75-8 | 500 |
| thiamine mononitrate* | CAS 532-43-4 | 1,1 |
| Riboflavin 5'-monophosphate sodium salt* | CAS 532-43-4 | 1,05 |
| Pyridoxal 5'-phosphate monohydrate* | CAS 41468-25-1 | 1,05 |
| Hydroxocobalamin acetat* | CAS 22465-48-1 | 0,0025 |
| Choline bitartrate* | CAS 87-67-2 | 82,5 |
| Selenomethionine* | CAS 3211-76-5 | 0,04125 |
| Zincorotate* | CAS 60388-02-5 | 7,5 |
| Managanese-(II)-Gluconate-Dihydrat* | CAS 6485-39- 8 | 1,5 |
| Copper Bisglycinate* | CAS 13479-54-4 | 0,75 |
| D-Alpha-Tocopheryl Acetate* | CAS 7695-91-2 | 9 |
| L-5-methyltetrahyd rofo late Magnafolate^{®} * | CAS 26560-38-3 | 0,7 |
| Potassium iodide * | CAS 7681-11-0 | 0,75 |
| ** Mixture C* | | |

**Table 8: Combination of Mixtures B+C+D**

| **Name of ingredients** | **Additional information about ingredients** | **Quantity (mg)** |
|---|---|---|
| Berberinchloride | CAS 633-65-8 | 500 |
| Monacolin K phyproof^{®} | CAS 75330-75-5 | 30 |
| Policosanol | CAS 142583-61-7 | 12 |
| Silymarin phyproof^{®} | CAS 33889-69-9 | 120 |
| L-Citrullin | CAS 372-75-8 | 500 |
| thiamine mononitrate* | CAS 532-43-4 | 1,1 |
| Riboflavin 5'-monophosphate sodium salt* | CAS 532-43-4 | 1,05 |
| Pyridoxal 5'-phosphate monohydrate* | CAS 41468-25-1 | 1,05 |
| Hydroxocobalamin acetat* | CAS 22465-48-1 | 0,0025 |
| Choline bitartrate* | CAS 87-67-2 | 82,5 |
| Selenomethionine* | CAS 3211-76-5 | 0,04125 |
| Zincorotate* | CAS 60388-02-5 | 7,5 |
| Managanese-(II)-Gluconate-Dihydrat* | CAS 6485-39- 8 | 1,5 |
| Copper Bisglycinate* | CAS 13479-54-4 | 0,75 |
| D-Alpha-Tocopheryl Acetate* | CAS 7695-91-2 | 9 |
| L-5-methyltetrahyd rofo late Magnafolate^{®} * | CAS 26560-38-3 | 0,7 |
| Potassium iodide * | CAS 7681-11-0 | 0,75 |
| Lactobacillus crispatus matrix protected 50 Mrd cfu/g ** | | 12 |
| Lactobacillus gasseri matrix protected 50 Mrd cfu/g ** | | 12 |
| Lactobacillus jensenii matrix protected 50 Mrd cfu/g ** | | 12 |
| Lactobacillus reuteri matrix protected 50 Mrd cfu/g ** | | 12 |
| Lactobacillus rhamnosus matrix protected 50 Mrd cfu/g ** | | 12 |
| Punica granatum extract ** | CAS 84961-57-9 | 200 |
| ** *Mixture D* | | |
| ** Mixture C* | | |

### Treatment

Study subjects receive oral doses of the compositions described above in the following combinations:
- Mixture A for 28-30 days
- Combination of Mixtures A+C for 28-30 days
- Combination of Mixtures B+C for 28-30 days
- Multiphase-Combination of Mixtures B+C for 28-30 days, Mixture D from Day 8 to Day 14

Mixture A will be administered as water-soluble powder, in two separate doses, twice per diem.

A combination of Mixtures A+C will be administered as water-soluble powder, in two separate doses, twice per diem.

A combination of Mixtures B+C will be administered in two capsules.

Mixture D will be administered in a separate capsule.

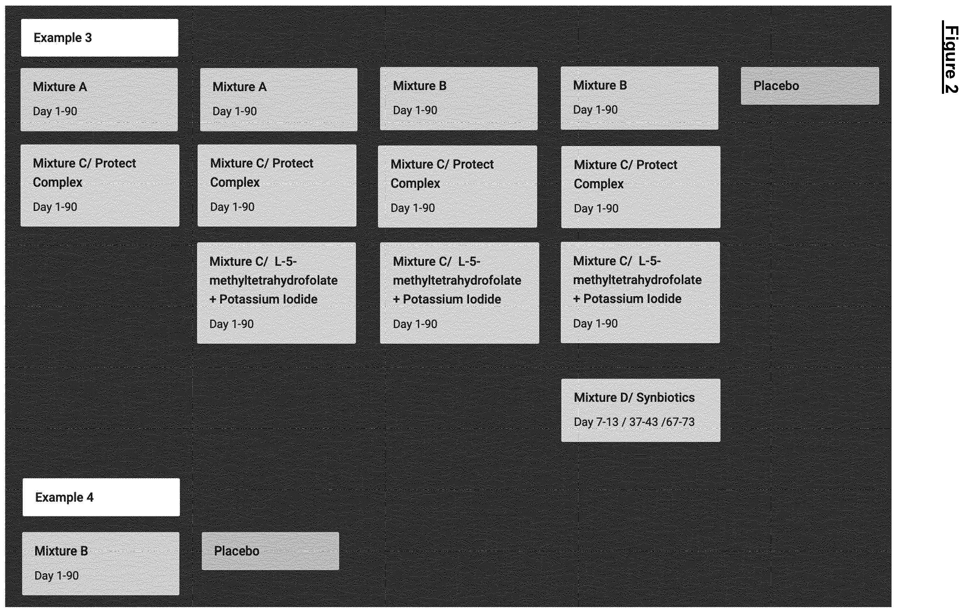

As control, a group of study subjects receive oral placebos. The treatment regimen is depicted in Fig. 2.

### Treatment Duration

### 3 months

### Study Design

Four women with regular menses (every 28-30 d) fulfilling below criteria are recruited for each research question. The mean body mass index (BMI) in the study population is comparable as are age, weight and ethnicity, following (Dunaif A, et al. Profound peripheral insulin resistance, independent of obesity, in polycystic ovary syndrome. Diabetes. 1989; 38:1165-74. [PubMed: 2670645]).

### Insulin Resistance

Insulin resistance is diagnosed using NIH criteria (1 Dunaif A. Insulin resistance and the polycystic ovary syndrome: mechanism and implications for pathogenesis. Endocr Rev. 1997; 18:774-800. [PubMed: 9408743]) and Rotterdam diagnostic criteria (2 Moran L, Teede H. Metabolic features of the reproductive phenotypes of polycystic ovary syndrome. Hum Reprod Update. 2009; 15:477-88. [PubMed: 19279045]). Thus, the study will include women with the most marked metabolic abnormalities - hyperandrogenism and chronic anovulation (i.e. NIH criteria) - independent of polycystic ovaries.

### PCOS

In PCOS a low SHBG concentration and low DHEAS level is an integrated marker of insulin resistance and may reflect insulin resistance more consistently than HOMA-IR itself. However, the low index of individuality of SHBG caused by the relatively higher inter- to intraindividual variation seen in PCOS suggests that it is unsuitable as a screening marker for insulin resistance alone, but, rather, that it is a marker for insulin resistance only after the diagnosis of PCOS. Once the diagnosis of PCOS is made, then a low SHBG concentration may be useful to identify those individuals with PCOS who are insulin resistant for targeted treatment with insulin sensitizing agents.

The mean body mass index (BMI) in the study population is comparable as are age, weight and ethnicity. No subject should be taking any medication at the start of the study or for the preceding 6 months, and there should be no concurrent illness. All subjects are on an unrestricted diet and are instructed not to modify their usual eating patterns during the period of sampling.

### Data Collection

Laboratory assays are performed at the beginning and at the end of the second and the third menstrual cycle.

Venous blood samples are collected from the antecubital vein between 8 and 9 am after an overnight 12-h fast. Moreover, glucose levels are measured in samples obtained 2 h after the oral ingestion of 75 g glucose. Plasma levels of glucose and serum levels of insulin are assayed by routine laboratory techniques using commercially available kits.

The homeostatic model for insulin resistance (HOMA1-IR) is calculated as follows: fasting glucose (mg/dL) × fasting insulin (mIU/L)/405.

Shortly after blood collection, all women will complete a questionnaire assessing demographic characteristics, smoking, physical activity, education, occupation, stress exposure, general health, number and duration of marriages, number of sexual partners, deliveries and abortions, as well as questionnaires measuring female sexual function and depressive symptoms.

Ovulation is assessed through ultrasound examination.

LH-Levels and ovulation will be assessed during mid-cycle using the at-home-ovulation predictor kit "Clearplan Easy Fertility Monitor (CPEFM)", assessing urinary hormone (LH) and estrone-3-glucuronide (E3G).

In addition, hormonal imbalance is determined by blood tests of reproductive hormone levels. Measuring follicle-stimulating hormone, luteinizing hormone (LH) and estrogen on day 3 and progesterone on day 21 (luteal phase) of a normal cycle can indicate whether the hormonal state is compatible with pregnancy. Abnormal LH or progesterone production may result in an abnormal monthly basal body temperature chart. If progesterone is low, basal body temperature may not increase during the second half of the cycle after ovulation. Without sufficient progesterone, the endometrium is not adequately prepared for implantation of an embryo.

The female sexual function index (FSFI) is a validated test evaluating all phases of the female sexual cycle, sexual satisfaction and dyspareunia in the last 4 weeks. It is composed of 19 items divided into 6 collective domains (subscales): sexual desire, sexual arousal, lubrication, orgasm, satisfaction and pain. The items are scored on a 5-point or 6-point scale, with responses ranging from 0 to 5 or 1 to 5 (0 indicates no sexual activity in the past month).

The presence and severity of depressive symptoms is evaluated using the Beck Depression Inventory-Second Edition (BDI-II).

Endometrial thickness will be assessed using a transvaginal transducer in days 6-10 of the menstrual cycle. The endometrial thickness is considered as the widest distance from the reflective interface between the endometrium and the myometrium of opposite sides on a sagittal view of the uterus. BMI was related to endometrial thickness both in PCOS women without IR and in women with IR and without PCOS suggests that both IR and anovulation are factors influencing BMI's correlation to endometrial thickness.

### Results

Mixture A, the combination of Mixture A + Mixture C, the Combination of Mixture B + Mixture C, and the Multiphase-Combination of Mixture B + Mixture C for 28-30 days, Mixture D from Day 7 to Day 13:
- improve ovulation,
- balance reproductive hormones as documented by changes in TSH, PRL, and 17-hydroxyprogesterone, SHBG, prolactin, testosterone and dehydroepiandrosterone sulphate,
- improve insulin parameters (LDL, HDL, triglycerides, IGT or IFT),
- decrease insulin resistance, as documented by HOMA1-IR,
   - induce changes in endometrium thickness,
   - decrease IGF-I,
- improve sexual functioning, as documented by changes in FSFI,
- influence cervical mucus and composition of vaginal flora (only for combination with Mixture D).

### EXAMPLE 4: Use of Mixture B in the treatment of decreased FSFI and low DHEA levels in women with PCOS using oral contraceptives

The following mixture is used in this example:

**Table 9: Mixture B**

| **Name of ingredients** | **Additional information about ingredients** | **Quantity (mg)** |
|---|---|---|
| Berberinchloride | CAS 633-65-8 | 500 |
| Monacolin K phyproof^{®} | CAS 75330-75-5 | 30 |
| Policosanol | CAS 142583-61-7 | 12 |
| Silymarin phyproof^{®} | CAS 33889-69-9 | 120 |
| L-Citrullin | CAS 372-75-8 | 500 |

### Treatment

Study subjects receive oral doses of the compositions described above. Mixture B will be administered in two capsules.

As control, a group of study subjects receives oral placebos. The treatment regimen is depicted in Fig. 2.

### Treatment Duration

### 3 months

### Study Design

Four women with regular menses (every 28-30 d) using an oral contraceptive (receiving a monophasic low-dose combined oral contraceptive pill of ethinyl estradiol and gestodene) with a >6 month history of decreased libido and low FSFI-scores are recruited for each research question. The mean body mass index (BMI) in the study population is comparable as are age, weight and ethnicity. No subject should be taking any medication in addition to the oral contraceptive pill at the start of the study or for the preceding 6 months, and there should be no concurrent illness. All subjects are on an unrestricted diet and are instructed not to modify their usual eating patterns during the period of sampling.

### Insulin resistance

Insulin resistance is diagnosed using NIH criteria (1 Dunaif A., Insulin resistance and the polycystic ovary syndrome: mechanism and implications for pathogenesis. Endocr Rev. 1997; 18:774-800. [PubMed: 9408743]) and Rotterdam diagnostic criteria (Moran L, and Teede H. Metabolic features of the reproductive phenotypes of polycystic ovary syndrome. Hum Reprod Update. 2009; 15:477-88. [PubMed: 19279045]). Thus, the study includes women with the most marked metabolic abnormalities - hyperandrogenism and chronic anovulation (i.e. NIH criteria) - independent of polycystic ovaries.

### DHEAS, ovary size and sexual function

Studies have shown a direct association between an endogenous level of DHEASS below the tenth percentile and low sexual responsiveness in women aged 45 years or older. In women aged 18-44 years, concentrations of DHEASS below the tenth percentile were directly associated with low sexual desire, arousal, and responsiveness.

Recent studies (Christodoulaki C. et.al. J Family Reprod Health. 2017; 11(1):24-29) have revealed a significant negative correlation of DHEAS-S with the mean volume of the right and left ovary and with the maximum volume of the largest ovary in women with PCOS. Thus, low libido and sexual function, resulting from low DHEAS levels, can be explained with hyper-stimulation of ovaries related to insulin resistance in women with PCOS. We conclude, that treatment of insulin resistance in women with clinical or subclinical PCOS will lead to an improvement in libido and sexual function.

### Data collection

Laboratory assays are performed at the beginning and at the end of the study. Venous blood samples are collected from the antecubital vein between 8 and 9 am after an overnight 12-h fast. Moreover, glucose levels are measured in samples obtained 2 h after the oral ingestion of 75 g glucose. Plasma levels of glucose and serum levels of insulin are assayed by routine laboratory techniques using commercially available kits.

The homeostatic model for insulin resistance (HOMA1-IR) is calculated as follows: fasting glucose (mg/dL) × fasting insulin (mIU/L)/405.

Shortly after blood collection, all women complete a questionnaire assessing demographic characteristics, smoking, physical activity, education, occupation, stress exposure, general health, number and duration of marriages, number of sexual partners, deliveries and abortions, as well as questionnaires measuring female sexual function and depressive symptoms.

The female sexual function index (FSFI) is a validated test evaluating all phases of the female sexual cycle, sexual satisfaction and dyspareunia in the last 4 weeks. It is composed of 19 items divided into 6 collective domains (subscales): sexual desire, sexual arousal, lubrication, orgasm, satisfaction and pain. The items are scored on a 5-point or 6-point scale, with responses ranging from 0 to 5 or 1 to 5 (0 indicates no sexual activity in the past month).

The presence and severity of depressive symptoms will be evaluated using the Beck Depression Inventory-Second Edition (BDI-II).

### Results

Mixture B improves symptoms associated with insulin resistance and decreased libido and specifically:
- increases dehydroepiandrosterone sulphate,
- improves insulin parameters (LDL, HDL, triglycerides, IGT or IFT),
- induces changes in FSFI: correlations between HOMA1-IR and desire or sexual satisfaction should be stronger than correlations between HOMA1-IR and the remaining domains assessed by FSFI,
- irrespective of the underlying condition, sexual functioning improves after insulin-sensitizing treatment.

## Claims

1. A composition comprising L-citrullin and silymarin; and
a. Mixture A comprising:
i. myo-inositol;
ii. D-chiro-inositol;
or
b. Mixture B comprising:
i. berberine;
ii. monacolin K; and
iii. policosanol.

2. The composition of claim 1, wherein the composition comprising Mixture A comprises 150 to 180mg Silymarin, 300 to 750mg L-citrullin, 3.900 to 4.100 mg myo-inositol, and 30 to 70 mg D-chiro-inositol.

3. The composition of claim 1 or 2, wherein the composition comprising Mixture B comprises 150 to 180mg Silymarin, 300 to 750mg L-citrullin, 400 to 600 mg berberine, 10 to 30 mg of a synthetic monacolin K compound or 200 to 250 mg red yeast rice extract, and 10 to 20 mg policosanol.

4. The composition of any one of claims 1 to 3, further comprising any one or more of thiamine mononitrate (vitamin B1), Riboflavin 5'-monophosphate sodium salt (vitamin B2), pyridoxal 5'-phosphate monohydrate (vitamin B6), hydroxocobalamin acetate (vitamin B12), choline bitartrate (vitamin B4), D-alpha-tocopherylacetate (vitamin E), selenomethionine, zinc orotate, manganese-(II)-gluconate-dihydrate, copper bisglycinate, potassium iodide and L-5-methyltetrahydrofolate.

5. The composition of any one of claims 1 to 4, further comprising 5-L-Hydroxytryptophan, and/or Vitex agnus-castus extract or agnusides, and/or Black cohosh extract or triterpene glycosides, preferably comprising 50 to 200 mg 5-L-Hydroxytryptophan, preferably about 100 mg, and/or 8-16mg Vitex agnus-castus, preferably 12mg, and/or and 4-10 mg Black cohosh extract, preferably about 7 mg.

6. The composition of any one of claims 1 to 5, further comprising the bacterial strains *L. crispatus, L. gasseri, L. jensenii, L. reuteri* and *L. rhamnosus,* and optionally further comprising 100 mg to 300 mg pomegranate extract, preferably 200 mg.

7. The composition of any one of claims 1 to 6, further comprising one or more pharmaceutically acceptable carriers.

8. The composition of any one of claims 1 to 7, wherein the composition is formulated in the form of a powder, preferably a water-soluble powder or a capsule.

9. The composition of any one of claims 1 to 8, for use in the treatment of a disease or disorder, preferably selected from the group consisting of polycystic ovary syndrome (PCOS), difficulties with ovulation, decreased female sexual function index (FSFI), menopause, premenstrual syndrome (PMS), insulin-resistance, obesity, dyslipidemia, coronary heart disease, metabolic syndrome, weight gain, and increased appetite, reduced sexual desire, and hyperandrogenemia (HA).

10. A dietary supplement comprising the composition of any one of claims 1 to 8.

11. The dietary supplement of claim 10, which is in the form of a powder, preferably a water-soluble powder, a tablet, a capsule, or a solid or semi-solid candy, or a combination of one or more dosage forms.

12. Use of the dietary supplement of claim 10 or 11, to reduce insulin-resistance, to increase the rate of ovulation, or to decrease hyperandrogenism, balance the menstrual cycle and hormones (prolactin, testosterone and dehydroepiandrosterone sulphate), or to improve the female sexual function index (FSFI), and/or reduce menopause-associated symptoms such as insulin resistance, polyphagia, vaginal dryness, weight gain, reduced sexual desire, and/or hormonal-related mood swings, and/or improve metabolic syndrome and hyperandrogenemia in female subjects.

13. A pharmaceutical preparation consisting of a number of separately packaged and individually removable daily dose units placed in a packaging unit and intended for oral administration for a period of at least 21 consecutive days, preferably at least 28 consecutive days, wherein said daily dose units comprise a composition comprising L-citrullin and silymarin and
a. Mixture A comprising:
i. myo-inositol;
ii. D-chiro-inositol;
or
b. Mixture B comprising:
i. berberine;
ii. monacolin K; and
iii. policosanol.

14. A multiphase product for enhanced female fertility, comprising at least 28 consecutive daily dose units of a composition comprising L-citrullin and silymarin and
a. Mixture A comprising:
i. myo-inositol;
ii. D-chiro-inositol;
or
b. Mixture B comprising:
i. berberine;
ii. monacolin K; and
iii. policosanol; and wherein
the full-cycle additionally comprises Mixture C, which is administered for 28 consecutive days and Mixture D, which is additionally administered during follicular phase for 6 consecutive days, preferably from day 8 to 13.

15. The multiphase product of claim 14, wherein the basic composition further comprises thiamine mononitrate (vitamin B1), Riboflavin 5'-monophosphate sodium salt (vitamin B2), pyridoxal 5'-phosphate monohydrate (vitamin B6), hydroxocobalamin acetate (vitamin B12), choline bitartrate (vitamin B4), D-alpha-tocopherylacetate (vitamin E), selenomethionine, zinc orotate, manganese-(II)-gluconate-dihydrate, copper bisglycinate, potassium iodide and L-5-methyltetrahydrofolate.
